(19) **Europäisches Patentamt / European Patent Office / Office européen des brevets**

(11) **EP 4 577 254 B1**

(12) **EUROPEAN PATENT SPECIFICATION**

(45) Date of publication and mention
of the grant of the patent:
**26.11.2025  Bulletin 2025/48**

(21) Application number: **24725239.8**

(22) Date of filing: **25.04.2024**

(51) International Patent Classification (IPC):
*A61L 15/46* (2006.01)    *A61L 15/60* (2006.01)
*A61F 13/05* (2024.01)    *B32B 5/02* (2006.01)
*B32B 7/09* (2019.01)    *B32B 7/12* (2006.01)
*B32B 29/00* (2006.01)

(52) Cooperative Patent Classification (CPC):
**A61L 15/46; A61F 13/05; A61L 15/60; B32B 5/022;**
**B32B 7/09; B32B 7/12; B32B 29/002;**
A61L 2300/104; B32B 2266/12; B32B 2307/7376

(86) International application number:
**PCT/GB2024/051086**

(87) International publication number:
**WO 2024/224076 (31.10.2024 Gazette 2024/44)**

(54) **WOUND DRESSING**

WUNDVERBAND

PANSEMENT POUR PLAIES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority:  **26.04.2023  US 202363498311 P**
**30.06.2023  GB 202310054**

(43) Date of publication of application:
**02.07.2025  Bulletin 2025/27**

(73) Proprietor: **ConvaTec Limited**
**Deeside, Flintshire CH5 2NU (GB)**

(72) Inventors:
• **BALLAMY, Lucy**
**Deeside Flintshire CH5 2NU (GB)**
• **BROWN, Natalie**
**Deeside Flintshire CH5 2NU (GB)**
• **DAVIES, Liam**
**Deeside Flintshire CH5 2NU (GB)**
• **KESTEVEN, Donna**
**Deeside Flintshire CH5 2NU (GB)**
• **GASKELL, Beth**
**Deeside Flintshire CH5 2NU (GB)**
• **EADE, Annchalee**
**Deeside Flintshire CH5 2NU (GB)**
• **GRIFFITHS, Emma**
**Deeside Flintshire CH5 2NU (GB)**
• **JELLIMAN, Louise**
**Deeside Flintshire CH5 2NU (GB)**
• **FORBES, Lucy**
**Deeside Flintshire CH5 2NU (GB)**

(74) Representative: **Wilson Gunn**
**Centurion House**
**129 Deansgate**
**Manchester M3 3WR (GB)**

(56) References cited:
**WO-A1-2021/239653    US-A1- 2021 146 000**
**US-A1- 2023 022 434**

• **PERCIVAL STEVEN L. ET AL: "Antiseptics for**
**treating infected wounds: Efficacy on biofilms**
**and effect of pH", CRITICAL REVIEWS IN**
**MICROBIOLOGY, vol. 42, no. 2, 26 August 2014**
**(2014-08-26), GB, pages 293 - 309, XP055814526,**
**ISSN: 1040-841X, DOI: 10.3109/**
**1040841X.2014.940495**

**Description**

Technical Field of the Invention

The present invention relates to a wound dressing. In particular, the invention concerns a negative pressure wound dressing and is defined in the appended claims.

Background to the Invention

Wound dressings are known and are generally suitable for treating a variety of wounds, including chronic and acute wound types, sch as infected wounds, venous ulcers, diabetic ulcers, burns and surgical wounds.

Wounds are often colonised by a variety of micro-organisms, some of which may cause infection. It is increasingly recognised that microbial populations living within a biofilm environment contribute to delayed healing and infection. Biofilms are comprised of exopolymeric substances that are produced by bacteria once they attach to a surface, and this helps to protect micro-organisms from immune cells and antimicrobial agents. Together, with the overuse of antibiotics and the associated increase in bacterial resistance, these factors are impacting the efficacy of antibiotics in the treatment of wound infection. Effective alternatives to antibiotics are, therefore, desirable.

Wound dressings typically comprise an adhesive skin contact layer for detachably adhering the dressing to a dermal surface, a backing layer, and an absorbent structure arranged between the backing layer and the adhesive skin contact layer. The absorbent structure typically comprises a layer in contact with a wound, referred to as a wound contact layer, and an absorbent layer arranged between the wound contact layer and the backing layer to prevent pooling of exudate at the wound site which could otherwise increase the risk of infection. In use, the wound contact layer absorbs wound exudate from a wound site. The exudate is then absorbed from the wound contact layer toward and into the absorbent layer, which typically has a greater absorbance capacity than the wound contact layer. Attempts to provide an effective treatment to prevent wound infection include associating the wound contact layer or the absorbent layer with an antimicrobial agent.

However, such attempts to incorporate an antimicrobial agent into a conventional wound dressing have not provided wholly satisfactory outcomes.

For example, where the antimicrobial agent is associated with an absorbent layer that is separated from the wound by a wound contact layer, the efficacy of the antimicrobial at the wound site is minimal, primarily because the absorbent layer is not in contact with the wound site due to the wound contact layer being arranged between the wound site and the absorbent layer.

As a further example, where the antimicrobial agent is associated with a conventional wound contact layer having a basis weight of around 70-80 g/m$^2$, a significant proportion of the antimicrobial agent is absorbed into the wound exudate and absorbed toward the absorbent layer, away from the wound site. Disadvantageously, this reduces the quantity of antimicrobial agent available at the wound site to prevent infection.

Thus, while these arrangements provide a minor antimicrobial effect at a wound site, further improvements need to be made.

A simple alternative would be to increase the quantity of antimicrobial agent to mitigate the quantity of antimicrobial agent absorbed into the wound exudate and absorbed away from the wound site. However, disadvantageously, this may cause undesirable side effects with prolonged use such as cytotoxicity, hypersensitivity reactions, skin staining and systemic effects.

US 2021/146000 A1 discloses a negative pressure wound dressing comprising a wound contact layer and a transmission layer. Silver ions are embedded in a loaded matrix which can be placed between the transmission layer and the wound contact layer.

It is an object of embodiments of th present invention to at least partially overcome or alleviate the above problems and/or to provide an improved wound dressing.

Summary of the Invention

According to a first aspect of the present invention there is provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, characterised in that the wound contact layer comprises multiple layers each formed of carboxyalkylcellulose, or a salt thereof, fibres.

The wound dressing is a negative pressure wound dressing. Advantageously, negative pressure facilitates wound healing through a number of mechanisms, including removal of excess exudate, reduction in periwound edema and increased perfusion. Combined with the physical forces exerted by the negative pressure which draw the wound edges together, this can result in improved wound outcomes.

Further advantageously, ionic silver eliminates, or at least significantly reduces the number of, microorganisms at a wound site. It has been found that the present invention provides for a 4-log reduction of the total viable cell count of microorganisms at a wound site within 48 hours of incubation. As such, the present invention is particularly advantageous as it reduces the total viable cell count of microorganisms at a wound site to a level below that established by regulatory agencies (for example the Food and Drug Administration (FDA) of the United States).

As such, the present invention provides a synergistic effect of improved wound outcomes, primarily due to the negative pressure generated at the wound site, and elimination of, or at least a significant reduction of, microorganisms at the wound site, primarily due to the wound contact layer comprising ionic silver.

The negative pressure wound dressing may comprise a port for connecting the aperture to a source of negative pressure. The port may be arranged for connection of a source of negative pressure above the backing layer of the negative pressure wound dressing. The port may be arranged to connect to medical tubing. The negative pressure wound dressing may comprise an airway for connecting the aperture to a source of negative pressure. The airway may be arranged for connection of a source of negative pressure away from the backing layer of the negative pressure wound dressing. Airways are more popular than ports for connecting to negative pressure sources, because they are lower-profile and connection away from the wound site is less likely to cause pain. The airway may be arranged to connect to medical tubing. The airway may comprise a luer lock connector to connect to a corresponding luer lock connector on medical tubing connected to a pump apparatus.

The backing layer may be provided with a port, or airway for connection to the conduit. The port or airway may be located in that part of the backing layer that overlies the absorbent structure but towards the periphery of the absorbent structure so that it is not directly in vertical alignment with the centre of the wound dressing (or the wound when in use). This assists in the spread of wound exudate across the full extent of the absorbent layer.

The backing layer of the wound dressing may be a bacterial and viral barrier layer which preferably resists the ingress of liquid and air but allows moisture vapour transmission. In this way the backing layer enhances the overall fluid handling capacity of the wound dressing by allowing for the escape of moisture vapour through the backing layer while enabling the application of negative pressure to the wound. The backing layer may be a layer having a Moisture Vapor Transmission Rate (MVTR) of at least about 10,000 g/m$^2$ per 24 hours or in the range of from about 10,000g/m$^2$ to about 50,000g/m$^2$ per 24 hours as measured by the method described in BS EN 13726-2 2002 "Test methods for primary wound dressings Part 2 Moisture vapour transmission rate of permeable film dressings". The backing layer may be in the form of a film of polyurethane.

The wound contact layer may be formed of gel-forming fibres. By gel-forming fibres is meant hygroscopic fibres which upon the uptake of wound exudate become moist slippery or gelatinous. The gel forming fibres can be of the type which retain their structural integrity on absorption of exudate or can be of the type which lose their fibrous form and become an amorphous or structureless gel. The gel-forming fibres may be carboxymethylcellulose fibres, chemically modified cellulosic fibres or alkyl sulphonate modified cellulosic fibres, or a combination thereof. Preferably, the gel forming fibres are carboxymethylcellulose fibres. The antimicrobial agent may be dispersed upon the gel-forming fibres. The gel-forming fibres may be impregnated, coated or treated with the antimicrobial agent comprising ionic silver.

The gel-forming fibres may have an absorbency of at least 1 gram 0.9% saline solution per gram of fibre (as measured by the free swell method).

The carboxyalkylcellulose, or a salt thereof, may be carboxymethylcellulose, carboxyethylcellulose, or a salt thereof, or a combination thereof. The salt may be a sodium salt or a silver salt, or a combination thereof. Beneficially, sodium and/or silver salts of carboxyalkylcellulose fibres each provide significant biocompatibility and antimicrobial efficacy, together with a favourable fibre strength, compared to other gel-forming fibres and salts.

The wound contact layer may be formed of sodium carboxymethylcellulose fibres. Advantageously, it has been found that in embodiments of the invention comprising a wound contact layer formed of sodium carboxymethylcellulose fibres, the wound dressing is particularly effective at eliminating microorganisms at a wound site compared to negative pressure wound dressings comprising a wound contact layer formed of non-gelling fibres.

Surprisingly, it has been found that in the invention, in particular in embodiments of the invention comprising sodium carboxymethylcellulose fibres, comprising (e.g., impregnated with, coated with, treated with, or having dispersed upon them) ionic silver, the availability of the ionic silver at the wound site is not diminished by absorption of wound exudate through the wound contact layer toward an absorbent layer. As such, the ionic silver is effectively held in the carboxyalkylcellulose, or a salt thereof, fibres, for example sodium carboxymethylcellulose fibres, of the wound contact layer when the wound dressing is in use. Advantageously, this maintains the availability of the ionic silver as an antimicrobial agent at the wound site such that antimicrobial activity at the wound site is maintained for prolonged periods. Beneficially, this means that the negative pressure wound dressing of the invention does not need to be replaced (so as to replenish the antimicrobial agent at the wound site) as regularly as wound dressings of the prior art.

Notably, the present invention, in particular in embodiments comprising sodium carboxymethylcellulose fibres, the efficacy of the ionic silver antimicrobial agent is not reduced despite the wound dressing of the present invention being a negative pressure wound dressing which, according to some theories in the prior art, may be considered to draw active agents incorporated into wound dressings away from a wound site and toward the source of negative pressure. Thus, the present invention exhibits advantages of reliable availability of the ionic silver as an antimicrobial agent at the wound site.

The antimicrobial agent may be dispersed upon the carboxyalkylcellulose fibres. The carboxyalkylcellulose fibres may be impregnated, coated or treated with the antimicrobial agent comprising ionic silver.

The carboxyalkylcellulose, or a salt thereof, fibres may have a degree of substitution of between about 0.05 carboxyalkyl groups per glucose unit and about 0.50 carboxyalkyl groups per glucose unit, between about 0.10 carboxyalkyl groups per glucose unit and about 0.50 carboxyalkylgroups per glucose unit, between about 0.15 carboxyalkyl groups per glucose unit and about 0.45 carboxyalkyl groups per glucose unit, between about 0.20 carboxyalkyl groups per glucose unit and about 0.40 carboxyalkyl groups per glucose unit, between about 0.25 carboxyalkyl groups per glucose unit and about 0.35 carboxyalkyl groups per glucose unit, or about 0.30 carboxyalkyl groups per glucose unit.

The carboxyalkylcellulose, or a salt thereof, fibres may have a degree of substitution of from about 0.05 carboxyalkyl groups per glucose unit to about 0.50 carboxyalkyl groups per glucose unit, from about 0.05 carboxyalkyl groups per glucose unit to about 0.40 carboxyalkyl groups per glucose unit, from about 0.05 carboxyalkyl groups per glucose unit to about 0.30 carboxyalkyl groups per glucose unit, from about 0.05 carboxyalkyl groups per glucose unit to about 0.20 carboxyalkyl groups per glucose unit, or from about 0.05 carboxyalkyl groups per glucose unit to about 0.10 carboxyalkyl groups per glucose unit.

The carboxyalkylcellulose, or a salt thereof, fibres may have a degree of substitution of from about 0.10 carboxyalkyl groups per glucose unit to about 0.50 carboxyalkyl groups per glucose unit, from about 0.10 carboxyalkyl groups per glucose unit to about 0.40 carboxyalkyl groups per glucose unit, from about 0.10 carboxyalkyl groups per glucose unit to about 0.30 carboxyalkyl groups per glucose unit, or from about 0.10 carboxyalkyl groups per glucose unit to about 0.20 carboxyalkyl groups per glucose unit.

The carboxyalkylcellulose, or a salt thereof, fibres may have a degree of substitution of from about 0.15 carboxyalkyl groups per glucose unit to about 0.50 carboxyalkyl groups per glucose unit, from about 0.15 carboxyalkyl groups per glucose unit to about 0.40 carboxyalkyl groups per glucose unit, from about 0.15 carboxyalkyl groups per glucose unit to about 0.30 carboxyalkyl groups per glucose unit, or from about 0.15 carboxyalkyl groups per glucose unit to about 0.20 carboxyalkyl groups per glucose unit.

The carboxyalkylcellulose, or a salt thereof, fibres may have a degree of substitution of from about 0.20 carboxyalkyl groups per glucose unit to about 0.50 carboxyalkyl groups per glucose unit, from about 0.20 carboxyalkyl groups per glucose unit to about 0.40 carboxyalkyl groups per glucose unit, or from about 0.20 carboxyalkyl groups per glucose unit to about 0.30 carboxyalkyl groups per glucose unit,.

The carboxyalkylcellulose, or a salt thereof, fibres may have a degree of substitution of from about 0.25 carboxyalkyl groups per glucose unit to about 0.50 carboxyalkyl groups per glucose unit, from about 0.25 carboxyalkyl groups per glucose unit to about 0.40 carboxyalkyl groups per glucose unit, or from about 0.25 carboxyalkyl groups per glucose unit to about 0.30 carboxyalkyl groups per glucose unit.

The carboxyalkylcellulose, or a salt thereof, fibres may have a degree of substitution of from about 0.30 carboxyalkyl groups per glucose unit to about 0.50 carboxyalkyl groups per glucose unit, or from about 0.30 carboxyalkyl groups per glucose unit to about 0.40 carboxyalkyl groups per glucose unit,.

The carboxyalkylcellulose, or a salt thereof, fibres may have a degree of substitution of from about 0.35 carboxyalkyl groups per glucose unit to about 0.50 carboxyalkyl groups per glucose unit, or from about 0.35 carboxyalkyl groups per glucose unit to about 0.40 carboxyalkyl groups per glucose unit.

The carboxyalkylcellulose, or a salt thereof, fibres may have a degree of substitution of from about 0.40 carboxyalkyl groups per glucose unit to about 0.50 carboxyalkyl groups per glucose unit, or from about 0.40 carboxyalkyl groups per glucose unit to about 0.45 carboxyalkyl groups per glucose unit.

The carboxyalkylcellulose, or a salt thereof, fibres may have a degree of substitution of from about 0.45 carboxyalkyl groups per glucose unit to about 0.50 carboxyalkyl groups per glucose unit.

In embodiments comprising carboxyalkylcellulose, or a salt thereof, fibres, the fibres may have a degree of substitution of about 0.05 carboxyalkyl groups per glucose unit, about 0.10, about 0.15, about 0.20, about 0.25, about 0.30, about 0.35, about 0.40, about 0.45, or about 0.50 carboxyalkyl groups per glucose unit.

In embodiments comprising carboxyalkylcellulose, or a salt thereof, fibres, the fibres may have a degree of substitution of at least about 0.05 carboxyalkyl groups per glucose unit, about 0.10, about 0.15, about 0.20, about 0.25, about 0.30, about 0.35, about 0.40, about 0.45, or at least about 0.50 carboxyalkyl groups per glucose unit.

In embodiments comprising carboxyalkylcellulose, or a salt thereof, fibres, the fibres may have a degree of substitution of no more than about 0.05 carboxyalkylgroups per glucose unit, about 0.10, about 0.15, about 0.20, about 0.25, about 0.30, about 0.35, about 0.40, about 0.45, or no more than about 0.50 carboxyalkylgroups per glucose unit.

The aforementioned degree of substitution of carboxyalkylcellulose, or a salt thereof, fibres, advantageously, provides a wound contact layer which is particularly effective at eliminating bacteria at a wound site and which comprises fibres that are relatively high-gelling, i.e., more absorbent and retentive, than fibres often comprised in wound contact layers of the prior art. As such, the wound contact layer is capable of absorbing and retaining a greater amount of wound exudate, without adversely affecting its adhesion to other layers within the absorbent structure, compared to wound contact layers which do not comprise fibres of carboxyalkylcellulose, or a salt thereof. Thus, pooling of wound exudate at the wound site is prevented, or at least significantly reduced.

Thus, in some embodiments there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres having a degree of substitution of between about 0.05 carboxyalkyl groups per glucose unit and about 0.50 carboxyalkyl groups per glucose unit.

In preferred embodiments, the wound contact layer comprises multiple layers each formed of carboxymethylcellulose fibres, in particular, sodium carboxymethylcellulose fibres.

Thus, in some embodiments there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of sodium carboxymethylcellulose fibres.

Thus, in some embodiments there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of sodium carboxymethylcellulose fibres having a degree of substitution of between about 0.05 carboxymethyl groups per glucose unit and about 0.50 carboxymethyl groups per glucose unit.

Thus, in some embodiments there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of sodium carboxymethylcellulose fibres having a degree of substitution of about 0.30 carboxymethyl groups per glucose unit.

The antimicrobial agent comprising ionic silver may be dispersed upon the sodium carboxymethylcellulose fibres and the sodium carboxymethylcellulose fibres may have a degree of substitution of between about 0.05 carboxymethyl groups per glucose unit and about 0.50 carboxymethyl groups per glucose unit, for example about 0.30 carboxymethyl groups per glucose unit.

The degree of substitution of the sodium carboxymethylcellulose fibres can be measured by IR spectroscopy (as defined in WO00/01425).

In embodiments comprising sodium carboxymethylcellulose fibres, the fibres may be made by carboxymethylating a woven or non-woven cellulosic fabric such that the absorbency is increased. The woven or non-woven cellulosic fabric may have an absorbency of between about 10g/g of physiological saline solution (Solution A, a sodium/calcium chloride solution described below) to about 30g/g of Solution A, about 15g/g of Solution A to about 25g/g of Solution A, about 15g/g of Solution A to about 20g/g of Solution A, or about 18g/g of Solution A, as measured by the method described in BS EN 13726-1 (2002) "Test methods for primary wound dressings", section 3.2 "Free swell absorptive capacity". The carboxymethylation is generally performed by contacting the fabric with an alkali and a carboxymethylating agent such as chloroacetic acid in an aqueous system.

Solution A, as described in BS EN 13726-1 (2002), consists of sodium chloride and calcium chloride solution containing 142 mmol of sodium ions and 2.5 mmol of calcium ions as the chloride salts. This solution has an ionic composition comparable to human serum or wound exudate. It is prepared by dissolving 8.298 g of sodium chloride and 0.368 g of calcium chloride dihydrate in deionised water and making up to 1 litre in a volumetric flask.

The wound contact layer may comprise cellulosic fibres. The wound contact layer may comprise a proportion of non-cellulosic textile fibres or gel forming fibres, or non-gel-forming fibres. The cellulosic fibres may be of known kind and may comprise continuous filament yarn and/or staple fibre. The fabric may comprise fibres textiled into a non-woven fabric.

The wound contact layer may comprise a combination or blend of gelling fibres (i.e., gel-forming fibres) and non-gelling fibres. The gelling fibres and the non-gelling fibres may be any such fibres disclosed herein. For example, the wound contact layer may comprise a combination or blend of fibres comprising a gelling fibre such as fibres formed of sodium carboxymethyl cellulose. The wound contact layer may comprise a combination or blend of fibres comprising a non-gelling fibre such as cellulose fibres.

The gelling fibres may be present in the combination or blend of fibres in an amount equal to about 10%, about 20%, about 30%, about 40%, about 50%, about 60%, about 70%, about 80%, or about 90% of the total mass of the combination or blend of fibres.

The non-gelling fibres may be present in the combination or blend of fibres in an amount equal to about 10%, about 20%, about 30%, about 40%, about 50%, about 60%, about 70%, about 80%, or about 90% of the total mass of the combination or blend of fibres.

The wound contact layer may be stitched using, cotton, nylon, polyester, any non-gelling fibre, or cellulose yarn, for example lyocell yarn. In particular, the wound contact layer may be stitched using lyocell yarn because, advantageously, this results in highly aligned cellulose fibres having a high degree of crystallinity, making the cellulose fibres collectively stronger so they are able to withstand manufacturing processes and provide a stronger wound contact layer which does not deteriorate in use. This also enhances the strength of the absorbent structure of the wound dressing.

The wound contact layer may, for example, be a Hydrofiber (RTM) material available from ConvaTec Ltd of Deeside.

The wound contact layer may have a basis weight of between about 160 g/m$^2$ and about 480 g/m$^2$, between about 160 g/m$^2$ and about 460 g/m$^2$, between about 160 g/m$^2$ and about 440 g/m$^2$, between about 160 g/m$^2$ and about 420 g/m$^2$, between about 160 g/m$^2$ and about 400 g/m$^2$, between about 160 g/m$^2$ and about 380 g/m$^2$, between about 160 g/m$^2$ and about 360 g/m$^2$, between about 160 g/m$^2$ and about 340 g/m$^2$, between about 160 g/m$^2$ and about 320 g/m$^2$, between about 160 g/m$^2$ and about 300 g/m$^2$, between about 160 g/m$^2$ and about 280 g/m$^2$, between about 160 g/m$^2$ and about 260 g/m$^2$, between about 160 g/m$^2$ and about 240 g/m$^2$, between about 180 g/m$^2$ and about 220 g/m$^2$, between about 190 g/m$^2$ and about 210 g/m$^2$, or about 200 g/m$^2$.

The wound contact layer may have a basis weight of about 160 g/m$^2$, about 170 g/m$^2$, about 180 g/m$^2$, about 190 g/m$^2$, about 200 g/m$^2$, about 210 g/m$^2$, about 220 g/m$^2$, about 230 g/m$^2$, about 240 g/m$^2$, about 250 g/m$^2$, about 260 g/m$^2$, about 270 g/m$^2$, about 280 g/m$^2$, about 290 g/m$^2$, about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, or about 480 g/m$^2$.

The wound contact layer may have a basis weight of at least about 160 g/m$^2$, about 170 g/m$^2$, about 180 g/m$^2$, about 190 g/m$^2$, about 200 g/m$^2$, about 210 g/m$^2$, about 220 g/m$^2$, about 230 g/m$^2$, about 240 g/m$^2$, about 250 g/m$^2$, about 260 g/m$^2$, about 270 g/m$^2$, about 280 g/m$^2$, about 290 g/m$^2$, about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, or at least about 480 g/m$^2$.

The wound contact layer may have a basis weight of no more than about 160 g/m$^2$, about 170 g/m$^2$, about 180 g/m$^2$, about 190 g/m$^2$, about 200 g/m$^2$, about 210 g/m$^2$, about 220 g/m$^2$, about 230 g/m$^2$, about 240 g/m$^2$, about 250 g/m$^2$, about 260 g/m$^2$, about 270 g/m$^2$, about 280 g/m$^2$, about 290 g/m$^2$, about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, or no more than about 480 g/m$^2$.

Advantageously, the wound contact layer may, therefore, have a basis weight which is greater than the basis weight of a typical wound contact layer of known wound dressings (i.e., around 70 to 100 g/m$^2$). This means that the concentration by area of the antimicrobial agent, ionic silver, according to the present invention, is increased relative to wound dressings of the prior art which comprise a wound contact layer having a basis weight of, for example, around 70 to 100 g/m$^2$.

By increasing the basis weight of the wound contact layer, relative to that of wound contact layers of the prior art, the concentration by area of ionic silver is increased without increasing the concentration by weight of ionic silver. Beneficially, this means that the antimicrobial effect of the negative pressure wound dressing of the invention can be achieved without increasing the concentration of ionic silver to levels which may present an adverse effect to the user, for example through cytotoxicity, hypersensitivity reactions, skin staining and systemic effects.

Advantageously, providing a wound contact layer comprising multiple layers, each formed of carboxyalkylcellulose, or a salt thereof, fibres is an efficient way to increase the basis weight of the wound contact layer. This is because, multiple layers of relatively low basis weight can be combined to provide a wound contact layer having an overall basis weight of, for example, between about 160 g/m$^2$ and about 480 g/m$^2$.

Each layer formed of carboxyalkylcellulose, or a salt thereof, fibres may have a basis weight of between about 40 g/m$^2$ and about 240 g/m$^2$, between about 50 g/m$^2$ and about 220 g/m$^2$, between about 60 g/m$^2$ and about 200 g/m$^2$, between about 70 g/m$^2$ and about 180 g/m$^2$, between about 80 g/m$^2$ and about 160 g/m$^2$, between about 90 g/m$^2$ and about 140 g/m$^2$, between about 90 g/m$^2$ and about 120 g/m$^2$, or about 100 g/m$^2$.

Each layer formed of carboxyalkylcellulose, or a salt thereof, fibres may have a basis weight of from about 40 g/m$^2$ to about 240 g/m$^2$, from about 40 g/m$^2$ to about 220 g/m$^2$, from about 40 g/m$^2$ to about 200 g/m$^2$, from about 40 g/m$^2$ to about 180 g/m$^2$, from about 40 g/m$^2$ to about 160 g/m$^2$, from about 40 g/m$^2$ to about 140 g/m$^2$, from about 40 g/m$^2$ to about 120 g/m$^2$, from about 40 g/m$^2$ to about 100 g/m$^2$, from about 40 g/m$^2$ to about 80 g/m$^2$, from about 40 g/m$^2$ to about 60 g/m$^2$, or from about 40 g/m$^2$ to about 50 g/m$^2$.

Each layer formed of carboxyalkylcellulose, or a salt thereof, fibres may have a basis weight of from about 60 g/m$^2$ to about 240 g/m$^2$, from about 60 g/m$^2$ to about 220 g/m$^2$, from about 60 g/m$^2$ to about 200 g/m$^2$, from about 60 g/m$^2$ to about 180 g/m$^2$, from about 60 g/m$^2$ to about 160 g/m$^2$, from about 60 g/m$^2$ to about 140 g/m$^2$, from about 60 g/m$^2$ to about 120 g/m$^2$, from about 60 g/m$^2$ to about 100 g/m$^2$, from about 60 g/m$^2$ to about 80 g/m$^2$, or from about 60 g/m$^2$ to about 70 g/m$^2$.

Each layer formed of carboxyalkylcellulose, or a salt thereof, fibres may have a basis weight of from about 100 g/m$^2$ to about 240 g/m$^2$, from about 100 g/m$^2$ to about 220 g/m$^2$, from about 100 g/m$^2$ to about 200 g/m$^2$, from about 100 g/m$^2$ to about 180 g/m$^2$, from about 100 g/m$^2$ to about 160 g/m$^2$, from about 100 g/m$^2$ to about 140 g/m$^2$, from about 100 g/m$^2$ to about 120 g/m$^2$, or from about 100 g/m$^2$ to about 110 g/m$^2$.

Each layer formed of carboxyalkylcellulose, or a salt thereof, fibres may have a basis weight of from about 140 g/m$^2$ to about 240 g/m$^2$, from about 140 g/m$^2$ to about 220 g/m$^2$, from about 140 g/m$^2$ to about 200 g/m$^2$, from about 140 g/m$^2$ to about 180 g/m$^2$, from about 140 g/m$^2$ to about 160 g/m$^2$, or from about 140 g/m$^2$ to about 150 g/m$^2$.

Each layer formed of carboxyalkylcellulose, or a salt thereof, fibres may have a basis weight of from about 180 g/m$^2$ to about 240 g/m$^2$, from about 180 g/m$^2$ to about 220 g/m$^2$, from about 180 g/m$^2$ to about 200 g/m$^2$, or from about 180 g/m$^2$ to about 190 g/m$^2$.

Each layer formed of carboxyalkylcellulose, or a salt thereof, fibres may have a basis weight of from about 200 g/m$^2$ to about 240 g/m$^2$, from about 200 g/m$^2$ to about 220 g/m$^2$, or from about 200 g/m$^2$ to about 210 g/m$^2$.

Each layer formed of carboxyalkylcellulose, or a salt thereof, fibres may have a basis weight of from about 220 g/m$^2$ to about 240 g/m$^2$, or from about 220 g/m$^2$ to about 230 g/m$^2$.

Each layer formed of carboxyalkylcellulose, or a salt thereof, fibres may have a basis weight of about 40 g/m$^2$, about 45 g/m$^2$, about 50 g/m$^2$, about 55 g/m$^2$, about 60 g/m$^2$, about 65 g/m$^2$, about 70 g/m$^2$, about 75 g/m$^2$, about 80 g/m$^2$, about 85 g/m$^2$, about 90 g/m$^2$, about 95 g/m$^2$, about 100 g/m$^2$, about 105 g/m$^2$, about 110 g/m$^2$, about 115 g/m$^2$, about 120 g/m$^2$, about 125 g/m$^2$, about 130 g/m$^2$, about 135 g/m$^2$, about 140 g/m$^2$, about 145 g/m$^2$, about 150 g/m$^2$, about 155 g/m$^2$, about 160 g/m$^2$, about 165 g/m$^2$, about 170 g/m$^2$, about 175 g/m$^2$, about 180 g/m$^2$, about 185 g/m$^2$, about 190 g/m$^2$, about 195 g/m$^2$, about 200 g/m$^2$, about 205 g/m$^2$, about 210 g/m$^2$, about 215 g/m$^2$, about 220 g/m$^2$, about 225 g/m$^2$, about 230 g/m$^2$, about 235 g/m$^2$, or about 240 g/m$^2$.

Thus, in some embodiments, there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres, further wherein each layer has a basis weight of between 80 g/m$^2$ and 160 g/m$^2$.

The combined basis weight of the multiple layers of carboxyalkylcellulose, or a salt thereof, fibres may be between about 160 g/m$^2$ and about 480 g/m$^2$, between about 160 g/m$^2$ and about 460 g/m$^2$, between about 160 g/m$^2$ and about 440 g/m$^2$, between about 160 g/m$^2$ and about 420 g/m$^2$, between about 160 g/m$^2$ and about 400 g/m$^2$, between about 160 g/m$^2$ and about 380 g/m$^2$, between about 160 g/m$^2$ and about 360 g/m$^2$, between about 160 g/m$^2$ and about 340 g/m$^2$, between about 160 g/m$^2$ and about 320 g/m$^2$, between about 160 g/m$^2$ and about 300 g/m$^2$, between about 160 g/m$^2$ and about 280 g/m$^2$, between about 160 g/m$^2$ and about 260 g/m$^2$ between about 160 g/m$^2$ and about 240 g/m$^2$, between about 180 g/m$^2$ and about 220 g/m$^2$, or about 200 g/m$^2$.

The combined basis weight of the multiple layers of carboxyalkylcellulose, or a salt thereof, fibres may be from about 160 g/m$^2$ to about 480 g/m$^2$, from about 160 g/m$^2$ to about 460 g/m$^2$, from about 160 g/m$^2$ to about 440 g/m$^2$, from about 160 g/m$^2$ to about 420 g/m$^2$, from about 160 g/m$^2$ to about 400 g/m$^2$, from about 160 g/m$^2$ to about 380 g/m$^2$, from about 160 g/m$^2$ to about 360 g/m$^2$, from about 160 g/m$^2$ to about 340 g/m$^2$, from about 160 g/m$^2$ to about 320 g/m$^2$, from about 160 g/m$^2$ to about 300 g/m$^2$, from about 160 g/m$^2$ to about 280 g/m$^2$, from about 160 g/m$^2$ to about 260 g/m$^2$, from about 160 g/m$^2$ to about 240 g/m$^2$, from about 160 g/m$^2$ to about 220 g/m$^2$, from about 160 g/m$^2$ to about 200 g/m$^2$, from about 160 g/m$^2$ to about 180 g/m$^2$, or from about 160 g/m$^2$ to about 170 g/m$^2$.

The combined basis weight of the multiple layers of carboxyalkylcellulose, or a salt thereof, fibres may be from about 200 g/m$^2$ to about 480 g/m$^2$, from about 200 g/m$^2$ to about 460 g/m$^2$, from about 200 g/m$^2$ to about 440 g/m$^2$, from about 200 g/m$^2$ to about 420 g/m$^2$, from about 200 g/m$^2$ to about 400 g/m$^2$, from about 200 g/m$^2$ to about 380 g/m$^2$, from about 200 g/m$^2$ to about 360 g/m$^2$, from about 200 g/m$^2$ to about 340 g/m$^2$, from about 200 g/m$^2$ to about 320 g/m$^2$, from about 200 g/m$^2$ to about 300 g/m$^2$, from about 200 g/m$^2$ to about 280 g/m$^2$, from about 200 g/m$^2$ to about 260 g/m$^2$, from about 200 g/m$^2$ to about 240 g/m$^2$, from about 200 g/m$^2$ to about 220 g/m$^2$, or from about 200 g/m$^2$ to about 210 g/m$^2$.

The combined basis weight of the multiple layers of carboxyalkylcellulose, or a salt thereof, fibres may be from about 240 g/m$^2$ to about 480 g/m$^2$, from about 240 g/m$^2$ to about 460 g/m$^2$, from about 240 g/m$^2$ to about 440 g/m$^2$, from about 240 g/m$^2$ to about 420 g/m$^2$, from about 240 g/m$^2$ to about 400 g/m$^2$, from about 240 g/m$^2$ to about 380 g/m$^2$, from about 240 g/m$^2$ to about 360 g/m$^2$, from about 240 g/m$^2$ to about 340 g/m$^2$, from about 240 g/m$^2$ to about 320 g/m$^2$, from about 240 g/m$^2$ to about 300 g/m$^2$, from about 240 g/m$^2$ to about 280 g/m$^2$, from about 240 g/m$^2$ to about 260 g/m$^2$, or from about 240 g/m$^2$ to about 250 g/m$^2$.

The combined basis weight of the multiple layers of carboxyalkylcellulose, or a salt thereof, fibres may be from about 300 g/m$^2$ to about 480 g/m$^2$, from about 300 g/m$^2$ to about 460 g/m$^2$, from about 300 g/m$^2$ to about 440 g/m$^2$, from about 300 g/m$^2$ to about 420 g/m$^2$, from about 300 g/m$^2$ to about 400 g/m$^2$, from about 300 g/m$^2$ to about 380 g/m$^2$, from about 300 g/m$^2$ to about 360 g/m$^2$, from about 300 g/m$^2$ to about 340 g/m$^2$, from about 300 g/m$^2$ to about 320 g/m$^2$, or from about 300 g/m$^2$ to about 310 g/m$^2$.

The combined basis weight of the multiple layers of carboxyalkylcellulose, or a salt thereof, fibres may be from about 340 g/m$^2$ to about 480 g/m$^2$, from about 340 g/m$^2$ to about 460 g/m$^2$, from about 340 g/m$^2$ to about 440 g/m$^2$, from about 340 g/m$^2$ to about 420 g/m$^2$, from about 340 g/m$^2$ to about 400 g/m$^2$, from about 340 g/m$^2$ to about 380 g/m$^2$, from about 340 g/m$^2$ to about 360 g/m$^2$, or from about 340 g/m$^2$ to about 350 g/m$^2$.

The combined basis weight of the multiple layers of carboxyalkylcellulose, or a salt thereof, fibres may be from about 400 g/m$^2$ to about 480 g/m$^2$, from about 400 g/m$^2$ to about 460 g/m$^2$, from about 400 g/m$^2$ to about 440 g/m$^2$, from about 400 g/m$^2$ to about 420 g/m$^2$, or from about 400 g/m$^2$ to about 410 g/m$^2$.

The combined basis weight of the multiple layers of carboxyalkylcellulose, or a salt thereof, fibres may be from about 420 g/m$^2$ to about 480 g/m$^2$, from about 420 g/m$^2$ to about 460 g/m$^2$, from about 420 g/m$^2$ to about 440 g/m$^2$, or from about 420 g/m$^2$ to about 430 g/m$^2$.

The combined basis weight of the multiple layers of carboxyalkylcellulose, or a salt thereof, fibres may be from about 440 g/m$^2$ to about 480 g/m$^2$, from about 440 g/m$^2$ to about 460 g/m$^2$, or from about 440 g/m$^2$ to about 450 g/m$^2$.

The combined basis weight of the multiple layers of carboxyalkylcellulose, or a salt thereof, fibres may be from about 460 g/m$^2$ to about 480 g/m$^2$, or from about 460 g/m$^2$ to about 470 g/m$^2$.

The combined basis weight of the multiple layers of carboxyalkylcellulose, or a salt thereof, fibres may be about 140 g/m$^2$, about 145 g/m$^2$, about 150 g/m$^2$, about 155 g/m$^2$, about 160 g/m$^2$, about 165 g/m$^2$, about 170 g/m$^2$, about 175 g/m$^2$, about 180 g/m$^2$, about 185 g/m$^2$, about 190 g/m$^2$, about 195 g/m$^2$, about 200 g/m$^2$, about 205 g/m$^2$, about 210 g/m$^2$, about 215 g/m$^2$, about 220 g/m$^2$, about 225 g/m$^2$, about 230 g/m$^2$, about 235 g/m$^2$, about 240 g/m$^2$, about 245 g/m$^2$, about 250 g/m$^2$, about 255 g/m$^2$, about 260 g/m$^2$, about 270 g/m$^2$, about 280 g/m$^2$, about 290 g/m$^2$, about 300 g/m$^2$, about 310 g/m$^2$, about 320 g/m$^2$, about 330 g/m$^2$, about 340 g/m$^2$, about 350 g/m$^2$, about 360 g/m$^2$, about 370 g/m$^2$, about 380 g/m$^2$, about 390 g/m$^2$, about 400 g/m$^2$, about 410 g/m$^2$, about 420 g/m$^2$, about 430 g/m$^2$, about 440 g/m$^2$, about 450 g/m$^2$, about 460 g/m$^2$, about 470 g/m$^2$, or about 480 g/m$^2$.

Thus, in some embodiments, there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres, further wherein the combined basis weight of the multiple layers is between 160 g/m$^2$ and 240 g/m$^2$.

The wound contact layer may comprise two, three, four, five, six, seven, eight, nine, ten, eleven, twelve, or more layers formed of carboxyalkylcellulose, or a salt thereof, fibres.

Thus, in some embodiments, there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres, further wherein the wound contact layer comprises only two layers.

In a preferred embodiment, the wound contact layer comprises two layers of carboxyalkylcellulose, or a salt thereof, fibres, each layer having a basis weight of about 100 g/m$^2$, therefore, the combined basis weight of the layers of the wound contact layer is about 200 g/m$^2$.

Thus, in some embodiments, there is provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres, further wherein the wound contact layer comprises only two layers, and further wherein each layer has a basis weight of 100 g/m$^2$.

At least two of the multiple layers of carboxyalkylcellulose, or a salt thereof, fibres may be consolidated by stitchbonding or lamination. The at least two layers may be consolidated using cellulose yarn, for example, lyocell yarn. Advantageously, this results in highly aligned cellulose fibres having a high degree of crystallinity, making the cellulose fibres collectively stronger so they are able to withstand manufacturing processes and provide a stronger wound contact layer which does not deteriorate in use. This also enhances the strength of the absorbent structure of the wound dressing.

Thus, in some embodiments, there is provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres, further wherein the multiple layers are consolidated by stitchbonding or lamination.

Thus, in some embodiments, there is provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises two layers each formed of carboxyalkylcellulose, or a salt thereof, fibres, further wherein the multiple layers are consolidated by stitchbonding.

The ionic silver may be bound to the polymeric backbone of the carboxyalkylcellulose, or a salt thereof, fibres.

The ionic silver may be in the form of any silver (Ag$^+$) salt such as silver nitrate, silver sulphate, silver chloride, silver sulphadiazine, or combinations thereof.

The ionic silver may be present in the antimicrobial agent at a concentration of from about 0.10 wt.% to about 4.0 wt.%, from about 0.10 wt.% to about 3.0 wt.%, from about 0.10 wt.% to about 2.0 wt.%, from about 0.10 wt.% to about 1.60 wt.%, from about 0.10 wt.% to about 1.40 wt.%, from about 0.10 wt.% to about 1.30 wt.%, from about 0.10 wt.% to about 1.20 wt.%, from about 0.10 wt.% to about 1.10 wt.%, from about 0.10 wt.% to about 1.00 wt.%, from about 0.10 wt.% to about 0.90 wt.%, or from about 0.10 wt.% to about 0.80 wt.%.

The ionic silver may be present in the antimicrobial agent at a concentration of from about 0.20 wt.% to about 4.0 wt.%, from about 0.20 wt.% to about 3.0 wt.%, from about 0.20 wt.% to about 2.0 wt.%, from about 0.20 wt.% to about 1.60 wt.%, from about 0.20 wt.% to about 1.40 wt.%, from about 0.20 wt.% to about 1.30 wt.%, from about 0.20 wt.% to about 1.20 wt.%, from about 0.20 wt.% to about 1.10 wt.%, from about 0.20 wt.% to about 1.00 wt.%, from about 0.20 wt.% to about 0.90 wt.%, or from about 0.20 wt.% to about 0.80 wt.%.

The ionic silver may be present in the antimicrobial agent at a concentration of from about 0.60 wt.% to about 3.0 wt.%, from about 0.60 wt.% to about 2.0 wt.%, from about 0.60 wt.% to about 1.60 wt.%, from about 0.60 wt.% to about 1.40 wt.%, from about 0.60 wt.% to about 1.30 wt.%, from about 0.60 wt.% to about 1.20 wt.%, from about 0.60 wt.% to about 1.10 wt.%, from about 0.60 wt.% to about 1.00 wt.%, from about 0.60 wt.% to about 0.90 wt.%, from about 0.60 wt.% to about 0.80 wt.%, from about 0.60 wt.% to about 0.70 wt.%, or from about 0.60 wt.% to about 0.65 wt.%.

The ionic silver may be present in the antimicrobial agent at a concentration of from about 0.80 wt.% to about 3.0 wt.%, from about 0.80 wt.% to about 2.0 wt.%, from about 0.80 wt.% to about 1.60 wt.%, from about 0.80 wt.% to about 1.40 wt.%, from about 0.80 wt.% to about 1.30 wt.%, from about 0.80 wt.% to about 1.20 wt.%, from about 0.80 wt.% to about 1.10 wt.%, from about 0.80 wt.% to about 1.00 wt.%, from about 0.80 wt.% to about 0.90 wt.%, or from about 0.80 wt.% to about 0.85 wt.%.

The ionic silver may be present in the antimicrobial agent at a concentration of from about 1.00 wt.% to about 3.0 wt.%, from about 1.00 wt.% to about 2.0 wt.%, from about 1.00 wt.% to about 1.60 wt.%, from about 1.00 wt.% to about 1.40 wt.%, from about 1.00 wt.% to about 1.30 wt.%, from about 1.00 wt.% to about 1.20 wt.%, from about 1.00 wt.% to about 1.10 wt.%, or from about 1.00 wt.% to about 1.05 wt.%.

The ionic silver may be present in the antimicrobial agent at a concentration of from about 1.20 wt.% to about 3.0 wt.%, from about 1.20 wt.% to about 2.0 wt.%, from about 1.20 wt.% to about 1.60 wt.%, from about 1.20 wt.% to about 1.40 wt.%, from about 1.20 wt.% to about 1.30 wt.%, or from about 1.20 wt.% to about 1.25 wt.%.

The ionic silver may be present in the antimicrobial agent at a concentration of from about from about 1.40 wt.% to about 4.0 wt.%, from about 1.40 wt.% to about 3.0 wt.%, from about 1.40 wt.% to about 2.0 wt.%, from about 1.40 wt.% to about 1.60 wt.%, or from about 1.40 wt.% to about 1.50 wt.%.

The ionic silver may be present in the antimicrobial agent at a concentration of from about 2.0 wt.% to about 5.0 wt.%, from about 2.0 wt.% to about 4.0 wt.%, or from about 2.0 wt.% to about 3.0 wt.%.

The ionic silver may be present in the antimicrobial agent at a concentration of from about 0.50 wt.% to about 1.8 wt. %, from about 0.60 wt.% to about 1.6 wt. %, from about 0.70 wt.% to about 1.5 wt. %, from about 0.80 wt.% to about 1.4 wt. %, from about 0.90 wt.% to about 1.35 wt. %, from about 0.95 wt.% to about 1.30. %, from about 1.00 wt.% to about 1.25 wt. %, from about 1.05 wt.% to about 1.25 wt. %, from about 1.10 wt.% to about 1.25 wt. %, from about 1.15 wt.% to about 1.25 wt. %, or about 1.20 wt.%.

The ionic silver may be present in the antimicrobial agent at a concentration of no more than about 0.10 wt.%, about 0.15 wt.%, about 0.20 wt.%, about 0.25 wt.%, about 0.30 wt.%, about 0.35 wt.%, about 0.40 wt.%, about 0.45 wt.%, about 0.50 wt.%, about 0.55 wt.%, about 0.60 wt.%, about 0.65 wt.%, about 0.70 wt.%, about 0.75 wt.%, about 0.80 wt.%, about 0.85 wt.%, about 0.90 wt.%, about 0.95 wt.%, about 1.00 wt.%, about 1.05 wt.%, about 1.10 wt.%, about 1.15 wt.%, about 1.20 wt.%, about 1.25 wt.%, about 1.30 wt.%, about 1.35 wt.%, about 1.40 wt.%, about 1.45 wt.%, about 1.50 wt.%, about 1.60 wt.%, about 1.70 wt.%, about 1.80 wt.%, about 1.90 wt.%, or no more than about 2.00 wt.%.

The wound contact layer may further comprise an antibiofilm agent. Advantageously, the efficacy of antimicrobial agents may be reduced by a biofilm matrix produced by bacteria at a wound site, therefore, strategies to disrupt the biofilm and expose micro-organisms within can be helpful in increasing the activity level of antimicrobial agents and thus reducing the concentration of such agents needed to make an effective composition.

Thus, in some embodiments, there may be provided a negative pressure wound dressing comprising a backing layer, an adhesive skin contact layer and an absorbent structure wherein the adhesive skin contact layer is configured to detachably adhere the dressing to a dermal surface, wherein the backing layer comprises an aperture for connection to a negative pressure source, wherein the absorbent structure comprises a wound contact layer arranged to contact the wound when the adhesive skin contact layer is adhered to the skin adjacent the wound, further wherein the wound contact layer comprises an antimicrobial agent comprising ionic silver, wherein the wound contact layer comprises multiple layers each formed of carboxyalkylcellulose, or a salt thereof, fibres, wherein the wound contact layer further comprises an antibiofilm agent.

The antibiofilm agent may comprise a chelating agent.

The chelating agent may comprise ethylenediaminetetra-acetic acid (EDTA) or a salt thereof. The salt of EDTA may be any di-, tri- or tetra-basic salt of EDTA, for example di-sodium salt, tri-sodium salt, tetra-sodium salt or calcium di-sodium salt, or combination thereof. Surprisingly, it has been found that a chelating agent, in particular, EDTA, is capable of effectively disrupting biofilms by chelating the metal ions, calcium and magnesium that maintain the integrity of the biofilm matrix. The presence of the chelating agent, for example EDTA, enhances the effect of the antimicrobial agent so that the concentration of antimicrobial agent, which is ionic silver, may be reduced and yet still achieve the required antimicrobial effect. By increasing the effectiveness of the antimicrobial agent, its concentration in the composition can be reduced thereby reducing the potential for adverse reactions.

The chelating agent may be present in an amount of between about 0.5 wt.% and about 10.0 wt.%, between about 0.6 wt.% and about 9.0 wt.%, between about 0.7 wt.% and about 8.0 wt.%, between about 0.8 wt.% and about 7.0 wt.%, between about 0.9 wt.% and about 6.0 wt.%, between about 1.0 wt.% and about 5.0 wt.%, between about 1.0 wt.% and about 5.0 wt.%, between about 1.0 wt.% and about 4.0 wt.%, between about 1.0 wt.% and about 3.0 wt.%, between about 1.5 wt.% and about 2.5 wt.%, or about 2.0 wt.%.

In embodiments comprising EDTA as a chelating agent, the EDTA may be present as the di-, tri- or tetra-basic salts of EDTA. It has been found that these salts are effective for eradicating microorganisms in the free floating or planktonic state and biofilm state alone or in the presence of an antimicrobial agent. For example, it has been found that EDTA at concentrations of 0.1-40 wt.% by volume was effective in killing a range of microorganisms both in the planktonic and biofilm state. Microorganisms that were effectively killed by EDTA included Pseudomonas aeruginosa, Serratia marcescens, vancomycin resistant Enterococcus (VRE) and methicillin resistant Staphylococcus aureus (MRSA).

The antimicrobial agent may comprise a buffering agent.

The buffering agent may be selected from the group comprising citric acid, di-sodium hydrogen phosphate, sodium citrate, acetic acid and sodium acetate, or combinations thereof.

The buffering agent may be present in an amount of about 1 wt.% to about 20 wt.%, about 4 wt.% to about 6 wt.%, or about 5 wt.% of the antimicrobial agent, so as to provide an isotonic composition.

The wound contact layer may comprise no more than about 1 wt.% silicone, about 0.8 wt.% silicone, about 0.6 wt.% silicone, about 0.4 wt.% silicone, no more than about 0.2 wt.% silicone, or about 0 wt.% silicone. Advantageously, in embodiments comprising a wound contact layer comprising no more than about 1 wt.% silicone, preferably 0 wt.% silicone, the antimicrobial has greater efficacy. This is because silicone acts as a barrier between the antimicrobial agent and the wound site. As such, in embodiments of the invention having a wound contact layer comprising no more than about 1 wt.% silicone, preferably 0 wt.% silicone, the barrier between the antimicrobial agent and the wound site is eliminated, or at least significantly reduced, and, therefore, the barrier between the antimicrobial agent and the wound site.

The wound contact layer may have a thickness of between about 1 mm and about 5 mm.

The adhesive skin contact layer may be a perforated mesh comprising perforations each having a diameter of between 1.25mm and 5.00mm. Advantageously, this means that the perforations have a sufficient diameter to provide breathability to the wound dressing in the event that some of the perforations reduce in diameter after their formation. As such, the adhesive skin contact layer maintains an environment optimised for wound healing.

The perforations comprise through holes in the adhesive skin contact layer which enable fluid to flow through the layer. Thus, in use, if the adhesive skin contact layer is arranged covering and in contact with a wound, the adhesive skin contact layer prevents, or at least substantially prevents, tissue ingrowth into other materials of the wound dressing.

Beneficially, the adhesive skin contact layer helps to maintain the integrity of the wound dressing while also creating an airtight, or substantially airtight, seal around the absorbent structure in order to maintain negative pressure at the wound.

The absorbent structure may be arranged between the backing layer and the adhesive skin contact layer. Advantageously, this means that wound exudate is absorbed by the absorbent structure before the exudate contacts the backing layer.

The adhesive skin contact layer may have a weight of between about 40gsm and about 250gsm, or about 200gsm.

Advantageously, an adhesive skin contact layer having a weight of between about 100gsm and about 250gsm provides a more malleable, gelled structure so, in the event of an air leak, the layer can seal the leak more easily than if the layer had a weight below 100gsm, in particular below 40gsm. Further, adhesive skin contact layers having a weight greater than 250gsm exhibit processing problems, for example the adhesive may 'flow out' from a cut shape, rendering the adhesive skin contact layer unusable.

The adhesive skin contact layer may comprise perforations each having a diameter of between about 1.75mm and about 4.00mm,.

The adhesive skin contact layer may have a thickness of between about 0.5mm and about 1.5mm.

The perforations may be formed by punch perforating, a hot pin process, a laser ablation process or an ultrasound process. **In** particular, the perforations may be formed by mechanical punch perforating. Advantageously, mechanical punch perforating prevents, or at least significantly reduces, formation of a doughnut or volcano structure around the perforations after their formation. The perforations may be formed by mechanical punch perforating an adhesive layer comprising a release liner covering at least one surface of the adhesive layer. Following mechanical punch perforating the adhesive layer, the release liner may be removed from the at least one surface of the adhesive layer to remove any 'slugs' that remain after mechanical punch perforating.

The adhesive skin contact layer may comprise a window. Thus, the adhesive skin contact layer may be an adhesive skin contact border layer having a perimetral shape. The window may be located central relative to the periphery of the adhesive skin contact layer.

The adhesive skin contact layer may comprise an upper surface and a lower surface. The upper surface and/or the lower surface may be flat, or at least substantially flat, for example owing to slight raised or lowered portions in the adhesive skin contact layer surrounding each perforation, such that the adhesive skin contact layer has an even, flat profile when viewed from a side.

At least a portion of the adhesive skin contact layer may be raised, or otherwise non-flatly shaped, such that the adhesive skin contact layer has an uneven or non-planar profile when viewed from a side.

The absorbent structure may comprise a superabsorbent layer.

The superabsorbent layer as defined in this specification is a layer comprising a superabsorbent material which can absorb and retain at least about 15 g/g 10min, when tested with Solution A.

Advantageously, the superabsorbent layer has a particularly favourable fluid management capacity which means that the superabsorbent layer absorbs and retains a significant volume of fluid (i.e., wound exudate). Advantageously, tests have shown that the superabsorbent layer has an absorbent capacity of around 25 g/g 10min, and at least about 15 g/g 10min, when tested with Solution A, and an absorbent capacity under compression -10.7 kPa (-80mmHg) negative pressure) of around 0.85 $g/cm^2$, and at least about 0.55 $g/cm^2$, when tested with Solution A.

Moreover, the superabsorbent layer is flexible such that it can easily conform to the contours of a patient's body at a wound site when in use, therefore enhancing comfort for the patient when wearing the wound dressing. Further, the superabsorbent layer maintains its structure when wet or dampened, therefore, the superabsorbent layer maintains its strength and integrity within the absorbent structure of the wound dressing, thus resisting dissociation and shredding of fibres when in use.

The superabsorbent layer may comprise an upper layer and a lower layer. The upper layer and the lower layer may enclose a first material comprising at least one of non-woven fibres, an absorbent material and a hot melt binder.

The hot melt binder may be present in an amount of between about 40 to about 100 $g/m^2$.

The superabsorbent layer may be capable of absorbing exudate from the wound contact layer and allowing the passage of fluid through it.

The absorbent material may be a superabsorbent material, in particular a superabsorbent material capable of absorbing wound exudate while allowing the passage of fluid through it. The superabsorbent material may be a powder, foam, sponge or fibre-based material. Advantageously, in embodiments where the superabsorbent material is a powder, the absorbent property of the superabsorbent material is enhanced due to a high surface area to volume ratio.

The non-woven fibres may be cellulose fibres. The cellulose non-woven fibres may be arranged as a cellulosic non-woven matrix of fibres. The cellulosic non-woven matrix may be a support onto which the absorbent material may be incorporated, for example the absorbent material may be dispersed upon the cellulosic non-woven matrix. The absorbent material may be dispersed evenly, or substantially evenly, throughout or upon the non-woven fibres, for example the cellulosic non-woven matrix. The absorbent material may be interspersed evenly, or substantially evenly, throughout the non-woven fibres, for example the cellulosic non-woven matrix.

The cellulose non-woven fibres may comprise solely of cellulosic fibre or may comprise a proportion of non-cellulosic textile fibre or gel forming fibre. The cellulosic fibre may comprise continuous filament yarn and/or staple fibre.

The upper layer and/or the lower layer may comprise cellulosic fibres. The upper layer and/or the lower layer may comprise fibres formed of a hot melt binder, for example EVA or PLA, in addition to or instead of the cellulosic fibres.

Advantageously, the first material, the upper layer and the lower layer of the superabsorbent layer exhibit structural integrity to the superabsorbent layer and, therefore, the integrity of the superabsorbent layer is maintained even when wet, for example when wet with exudate.

The first material may comprise gel-forming fibres. Gel-forming fibres are fibres which, upon the uptake of wound exudate, become moist, slippery and/or gelatinous and thus reduce the tendency for the surrounding fibres to adhere to the wound. The gel-forming fibres may be hygroscopic. Beneficially, the gel-forming fibres prevent, or at least significantly prevent, the superabsorbent layer adhering to the wound in use, which may be uncomfortable for the patient, especially when the wound dressing is removed to be replaced by a fresh wound dressing.

The gel forming fibres may be of the type which retain their structural integrity on absorption of fluid (e.g., wound exudate) or may be of the type which lose their fibrous form and become an amorphous or structureless gel.

The gel-forming fibres may be sodium carboxymethylcellulose fibres, chemically modified cellulosic fibres, alkyl sulphonate modified cellulosic fibres such as those described in WO2012/061225, pectin fibres, alginate fibres, chitosan fibres, hyaluronic acid fibres, or other polysaccharide fibres or fibres derived from gums. The cellulosic fibres preferably have a degree of substitution of at least 0.05 carboxymethyl groups per glucose unit. The gel forming fibres preferably have an absorbency of at least 2 grams 0.9% saline solution per gram of fibre (as measured by the free swell method).

In embodiments where the superabsorbent layer comprises gel-forming fibres, the gel-forming fibres may comprise the hot melt binder.

The superabsorbent layer may have a basis weight of between about 300 $g/m^2$ and about 550 $g/m^2$.

The superabsorbent layer may have a thickness of between about 1.40mm and about 4.00mm.

The thickness of the superabsorbent layer may be measured using a Hampden Soft Materials Thickness Gauge. The method of measuring the thickness of the superabsorbent layer using a Hampden Soft Materials Thickness Gauge may include:

a. Raising the foot of the thickness gauge high enough to place a test sample beneath it;
b. Lowering the foot onto the test sample until an indicator light comes on;
c. Leaving the foot in contact with the test sample for 10 seconds from when the indicator light came on. If the light has remained on, record the reading;
d. Repeat for the required number of replicates (for example 10 replicates) ensuring the gauge is zeroed in between each replicate.

The superabsorbent layer may have a density of between about 0.200 $g/cm^3$ and about 0.305 $g/cm^3$.

The superabsorbent layer may have a tensile strength (dry) of between about 30 N/50mm and about 60 N/50mm.

Advantageously, a superabsorbent layer having a tensile strength as described above means that the superabsorbent layer is flexible and conforms well to the contours of a patient's body at the wound site when in use.

The superabsorbent layer may have an absorbent capacity of between about 15 g/g 10min and about 35 g/g 10min.

Advantageously, a superabsorbent layer having an absorbent capacity of between about 15 g/g 10min and about 35 g/g 10min, in particular about 25 g/g 10min, means that the superabsorbent layer absorbs about 1g / $cm^2$, which is considerably more favourable than absorbent layers used in known wound dressings.

The absorbent capacity of the superabsorbent layer may be measured using the Liquid Handling Direct Immersion Technique, as follows:

GSM

a. Cut a sample to be tested to a 5cm x 5cm square (A1 = 25cm$^2$);
b. Weigh each of the samples in grams;
c. Calculate:

$$GSM = \frac{(\text{sample weight(g)})}{0.0025\text{m}^2} = \text{g/m}^2$$

Liquid Handling

a. Place the dressing test pieces in filter paper bags and weigh the sample in grams (W1);
b. Add 40 times the weight of the sample in warmed (37 °C $\pm$ 1 °C) hydrating fluid (solution A);
c. Incubate at 37°C ($\pm$ 2°C) for 30 ($\pm$ 1) minutes;
d. Using forceps remove the sample(s) and empty filter paper bag(s) from the incubator and suspend (holding the filter paper bags by one corner) over the dish for 30 seconds;
e. Re-weigh the sample(s) (W2);
f. Repeat above steps with the 3 empty filter paper bags, use to calculate the mean fluid uptake by the filter paper bags and subtract from weight of samples after hydration;
g. Calculate:

Weight of solution absorbed per g of sample: $\dfrac{(W2 - W1)}{W1}$ g/g

And Liquid uptake per unit area: $\dfrac{(W2 - W1)}{A1}$ g/cm$^2$

Fluid Retention

a. Following step (e) above, remove sample from balance with forceps and place flat on dry perforated stainless-steel plate;
b. Immediately cover the wet sample with a 40mmHg weight (1359.51g for a 5x5cm);
c. Leave in place for 1 minute, and then remove the weight;
d. Remove the sample from the perforated plate and weigh (W3);
e. Repeat with empty filter paper bags and use to calculate mean fluid retained by the filter paper bags and subtract from weight of samples after;
f. Calculate:

Weight of solution retained per g of sample: $\dfrac{(W3 - W1)}{W1}$ g/g

And Liquid retention per unit area: $\dfrac{(W3 - W1)}{A1}$ g/cm$^2$

The superabsorbent layer may have an absorbency under compression capacity of between about 0.50 g/cm$^2$ and about 1.20 g/cm$^2$.

Absorbency under compression may be measured using the following test:

1. Cut a sample to be tested to 5cm x 5cm squares (A1 = 25cm$^2$) (test sample);
2. Place a test sample into filter paper bags and weigh each bag; record the weight in grams (W1);
3. Place the test sample onto a dry stainless-steel perforated plate, then place a weight equivalent to 80mmHg on each sample;
4. Repeat with three empty filter bags;
5. Add sufficient hydrating fluid (Solution A) at 20°C $\pm$ 2°C such that the stainless-steel perforated plate is covered and leave for 24 hours at 20°C ± 2°C;

6. Drain off the hydrating fluid (Solution A) and remove the combined weight;

7. Remove the test sample using forceps and weigh it immediately (W2);

8. Place the test sample onto a dry stainless steel perforated plate and then place a weight equivalent to 80mmHg onto the sample such that this load is applied evenly over the surface of the test sample;

9. Leave this in place for 15 minutes;

10. Reweigh the test sample in grams (W3);

11. Repeat above steps with the 3 empty filter paper bags, use to calculate the mean fluid retained by the filter paper bags and subtract from weight of samples after hydration;

12. Calculate:

> a. Weight of solution absorbed per g of sample: (W2 -W1)/W1 g/g
> b. Weight of fluid retained per g of sample: (W3 -W1)/W1 g/g
> c. Fluid absorbed unit area: (W2 -W1)/A1 g/cm$^2$
> d. Retained retention per unit area: (W3 -W1)/A1 g/cm$^2$
> e. % Fluid immobilised: (W3/W2)x100

The above described test mimics the compression of the structure which is expected when a -10.7 kPa (-80mmHg) negative pressure is applied to the wound site, as is the typical pressure used in negative pressure wound dressings.

Advantageously, a superabsorbent layer having an absorbency under compression value as above sufficiently absorbs fluid, (i.e., wound exudate) from the wound site such that the superabsorbent layer maintains an environment optimised for wound healing at the wound site.

The superabsorbent layer may be planar, or substantially planar.

The superabsorbent layer may comprise a plurality of superabsorbent layers, each layer comprising an upper layer and a lower layer, together which enclose a first material comprising non-woven fibres, an absorbent material and a hot melt binder.

The absorbent structure may comprise a transmission layer.

In embodiments comprising a superabsorbent layer, the transmission layer may be arranged between the wound contact layer and the superabsorbent layer.

The wound contact layer may comprise a first surface and a second surface. In use, the first surface may be inwardly facing toward the patient's body, and the second surface may be outwardly facing away from the patient's body. The first surface of the wound contact layer may contact the wound when the wound dressing is adhered to the skin adjacent to the wound (i.e., when in use). The second surface of the wound contact layer may be adjacent, and in contact with, a first surface of the transmission layer or the superabsorbent layer.

The transmission layer may comprise a first surface and a second surface. In use, the first surface may be inwardly facing toward the patient's body, and the second surface may be outwardly facing away from the patient's body.

The superabsorbent layer may comprise a first surface and a second surface. In use, the first surface may be inwardly facing toward the patient's body, and the second surface may be outwardly facing away from the patient's body.

The adhesive skin contact layer may provide an adhesive border. Advantageously, this means the adhesive skin contact layer easily allows the wound dressing to detachably adhere to a peri-wound dermal surface, and not adhere directly to a wound which may cause discomfort for a user.

The adhesive skin contact layer may be configured to detachably adhere the dressing to a peri-wound dermal surface.

The adhesive skin contact layer may comprise a window. Advantageously, the window allows the adhesive skin contact layer to be adhered around a wound, such that the wound site is placed within the window, therefore, allowing the patient's wound to be in contact, or be in fluid communication with, for example and preferably, the wound contact layer when the wound dressing is applied to the skin of a patient.

The adhesive skin contact layer may overlap the absorbent structure.

Each of the wound contact layer, the transmission layer and the superabsorbent layer may be laminated together. Advantageously, this means that the absorbent structure of the present invention is manufactured without requiring any relatively rigid dressing and binding components. Thus, the present invention provides an absorbent structure which is flexible, therefore benefits user comfort, and which is cheaper to manufacture than absorbent structures and, therefore negative pressure wound dressings, of the prior art. Moreover, lamination does not require any complex equipment or manufacturing techniques. Thus, the present invention is simple to manufacture.

The at least two of the wound contact layer, the transmission layer and the superabsorbent layer may be laminated by a laminator having a heating zone temperature between about 50°C and about 200°C.

The at least two of the wound contact layer, the transmission layer and the superabsorbent layer may be laminated by a laminator having a cooling zone temperature of between about 0°C and about 50°C.

The at least two of the wound contact layer, the transmission layer and the superabsorbent layer may be laminated by a laminator having a web tension (for each unwind reel) of between about 10 N and about 240 N.

The at least two of the wound contact layer, the transmission layer and the superabsorbent layer may be laminated together by a scatter-coat adhesive. The scatter-coat adhesive may be a hot melt, scatter-coat adhesive.

The scatter-coat adhesive may be polylactic acid, polycaprolactone, polyethylene (LDPE, HDPE), polypropylene, polyester, polyamide, ethylene vinyl acetate or polyurethane. Advantageously, each of these materials are cheap and easily obtained.

The scatter-coat adhesive may have a melt flow index of between about 5.2 g/10min and about 11.3 g/10min, as tested with 2.16 kg, 1" PVC die at 160°C.

The scatter-coat adhesive may have a maximum water content of between about 0.25% and about 0.45%.

The scatter-coat adhesive may comprise particles of which at least 98% have a particle size less than 600 $\mu$m, as measured by DIN EN ISO 4610.

The scatter-coat adhesive may comprise particles of which at least 98% have a particle size of between about 0.05 mm and about 0.80 mm.

The scatter-coat adhesive may have a mean molecular weight of between about 35,000 g/mol and about 65,000 g/mol.

The scatter-coat adhesive may have a melting point of between about 55°C and about 65°C.

The scatter-coat adhesive may have a solubility parameter of 9.30 cal/cm$^3$ to 9.45 cal/cm$^3$, for example of 9.34 cal/cm$^3$ to 9.43 cal/cm$^3$.

The wound contact layer may be stich bonded to the transmission layer or to the superabsorbent layer. The wound contact layer may be stitch bonded to the layer of the absorbent structure which is adjacent to, and in contact with, the second surface of the wound contact layer. In particular, the wound contact layer may be stitch bonded to the transmission layer and this, advantageously, increases the bond strength between the wound contact layer and the transmission layer. Further advantageously, the process of stitch bonding provides a plurality of holes in the layer(s) which is/are stitch bonded, and the holes facilitate the application of negative pressure to the wound site.

These advantageous effects are particularly advantageous in embodiments where the wound contact layer comprises Hydrofiber (RTM) obtainable from ConvaTec Limited of Deeside, UK or is formed of sodium carboxymethylcellulose fibres having a degree of substitution of between about 0.05 carboxymethyl groups per glucose unit and about 0.50 carboxymethyl groups per glucose unit, as described herein.

The transmission layer may be laminated or stitchbonded on a first surface to the wound contact layer and may be laminated or stitchbonded on a second surface to the superabsorbent layer.

The transmission layer may be arranged between the wound contact layer and the superabsorbent layer. Advantageously, this arrangement increases the absorbency capacity of the wound dressing and increases the amount of vapour transmitted by the wound dressing.

The transmission layer may be laminated on a first surface to the wound contact layer and on a second surface to the superabsorbent layer. Beneficially, this means that the transmission layer is adhered to the wound contact layer and to the superabsorbent layer without using relatively rigid dressing and binding components. The manufacture of the absorbent structure is therefore cheaper and simpler to manufacture, and provides for a flexible and, therefore, more comfortable wound dressing compared to that of the prior art.

The transmission layer may have a thickness of between about 1.0 mm and about 5.0 mm.

Advantageously, a transmission layer of relatively thin size, as described above, provides for a wound dressing of reduced thickness compared to wound dressings of the prior art. Thus, the wound dressing of the invention is flatter than many known wound dressings, and is therefore more comfortable for a user than known wound dressings having greater thickness.

The transmission layer may be formed of a polyurethane foam, a polyester foam, a hydrophilic polyurethane, silicone, gelatine, polypropylene, nitrile or EVA foams.

The superabsorbent layer may include any one or more of the features, optional or otherwise, of the invention according to any other aspect.

The wound contact layer may include any one or more of the features, optional or otherwise, of the invention according to any other aspect.

Advantageously, in embodiments comprising the transmission layer arranged between the wound contact layer and the superabsorbent layer, and in particular in embodiments where the wound contact layer comprises multiple layers each formed of sodium carboxymethylcellulose fibres having a degree of substitution of between about 0.05 carboxymethyl groups per glucose unit and about 0.50 carboxymethyl groups per glucose unit, the wound dressing of the invention prevents, or at least significantly reduces, lateral spread of wound exudate at the wound contact layer. As such, wound exudate initially wicks upwardly through the wound contact layer, and then laterally across the transmission layer before being absorbed by the superabsorbent layer. Thus, providing an improved wicking and movement of fluid away from the wound site. Beneficially, this arrangement is more efficient at removing wound exudate from a wound site, therefore, rendering the wound site more hygienic and increasing comfort for the user. Further beneficially, this arrangement increases the ability of the wound dressing to transmit vapour, therefore, facilitating the evaporation of fluid comprised in the wound exudate.

According to a second aspect of the present invention, there is provided a method for manufacturing a negative pressure wound dressing according to the first aspect of the invention comprising the steps:

a. Providing an adhesive skin contact layer and a backing layer;
b. Providing a wound contact layer and an absorbent structure comprising a transmission layer and a superabsorbent layer, wherein the wound contact layer comprises an antimicrobial agent comprising ionic silver, further wherein the wound contact layer comprises multiple layers each formed of carboxyalkylcellulose, or a salt thereof, fibres; and
c. Arranging the absorbent structure between the wound contact layer and the backing layer.

The invention according to the second aspect may of course include any one or more of the features, optional or otherwise, of the invention according to the first or third aspect.

According to a third aspect of the invention, there is provided a negative pressure wound exudate management system comprising the wound dressing of the broad aspect or the first aspect, and further comprising:

a source of negative pressure; and
a conduit or tube for providing negative pressure to the wound dressing via an internal lumen of the conduit or tube and the aperture in the wound dressing.

The source of negative pressure may comprise a pump for generating negative pressure and the tube may connect the pump and wound dressing.

The source of negative pressure may be capable of generating a minimum of 5.3 kPa (40mmHg) and a maximum of 26.7 kPa (200 mHg) for example 16.7 kPa (125mmHg), at the wound site.

The source of negative pressure may further comprise a one-way valve in-line between the pump and the wound dressing to maintain a negative pressure within the wound dressing when the pump is disconnected from the tube. Normal operation of the pump may include the generation of negative pressure, alternating periods of negative pressure generation, and/or no generation of negative pressure.

The one-way valve may be connected to one of the pump and the tube.

The conduit or tube may comprise a proximal end having an opening. The proximal end of the conduit or tube may be adhered around the opening in the backing layer of the wound dressing such that the opening in the backing layer is in fluid communication with the opening in the proximal end of the conduit or tube. The adhesion between the proximal end of the conduit or tube and the opening in the backing layer may for an airtight, or substantially airtight, seal between the conduit or tube and the backing layer.

The invention according to the third aspect may of course include any one or more of the features, optional or otherwise, of the invention according to any previous aspect.

Also disclosed is a method of using a negative pressure wound dressing including:

a. Providing a negative pressure wound dressing, a source of negative pressure and a conduit or tube for providing negative pressure to the wound dressing via an internal lumen of the conduit or tube;
b. Connecting the conduit at a first end to the source of negative pressure, and at a second end to a port or airway in a backing layer of the negative pressure wound dressing;
c. Securing the negative pressure wound dressing to the skin surrounding a wound;
d. Activating the source of negative pressure to apply a negative pressure to the wound.

The negative pressure wound dressing may comprise an adhesive skin contact layer. In step (c), the adhesive skin contact layer may be used to secure the wound dressing to the skin surrounding the wound.

The negative pressure wound dressing may be a negative pressure wound dressing according to the first aspect of the invention.

Detailed Description of the Invention

In order that the invention may be more clearly understood one or more embodiments thereof will now be described, by way of example only, with reference to the accompanying drawings, of which:

Figure 1       is an exploded view of a first embodiment of a negative pressure wound dressing;

Figure 2     is a schematic representation of a wound exudate management system according to some embodiments;

Figure 3     is a schematic showing an arrangement for performing Assessment 1;

Figure 4     is a chart showing Total Viable Cell Count of Example 1;

Figure 5     is a chart showing Total Viable Cell Count of Example 2;

Figure 6     is a chart showing Total Viable Cell Count of Example 3

Figure 7     is a chart showing Total Viable Cell Count of Example 4;

Figure 8     is a chart showing Total Viable Cell Count of Example 5;

Figure 9     is a chart showing Total Viable Cell Count of Example 6;

Figure 10    is a chart showing Total Viable Cell Count of Example 7;

Figure 11    is a chart showing Total Viable Cell Count of Example 8;

Figure 12    is a chart showing Total Viable Cell Count of Example 9;

Figure 13    is a chart showing Total Viable Cell Count of Example 10;

Figure 14    is a chart showing Total Viable Cell Count of Example 11;

Figure 15    is a chart showing Total Viable Cell Count of a polycarbonate coupon of Example 12;

Figure 16    is a chart showing Total Viable Cell Count of a wound dressing of Example 12;

Figure 17    is a chart showing Total Viable Cell Count of a polycarbonate coupon of Example 13;

Figure 18    is a chart showing Total Viable Cell Count of a wound dressing of Example 13;

Figure 19    is a chart showing Total Viable Cell Count of a polycarbonate coupon of Example 14; and

Figure 20    is a chart showing Total Viable Cell Count of a wound dressing of Example 14.

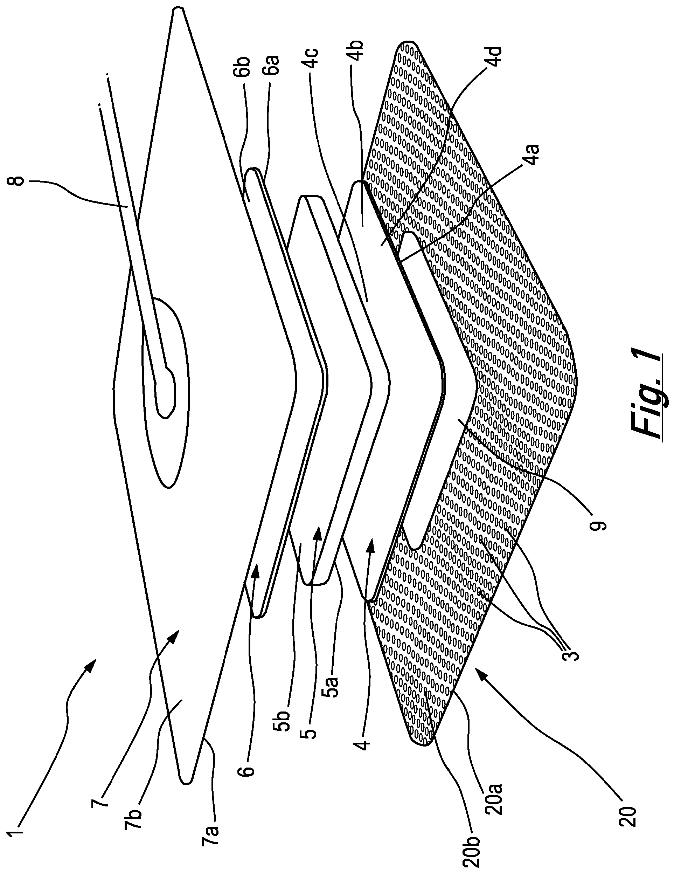

With reference to Figure 1, there is shown a negative pressure wound dressing 1.
The wound dressing 1 comprises an adhesive skin contact layer 20, a wound contact layer 4, a transmission layer 5, a superabsorbent layer 6, a backing layer 7 and an airway 8.
The adhesive skin contact layer 20 may have a perimetral shape with a central window 9, therefore, the adhesive skin contact layer 20 may be an adhesive skin contact border layer 20. The border layer 20 may comprise an outer periphery which defines the outer shape of the border layer 20, which, in this embodiment, is a square. The border layer 20 may comprise an inner periphery which defines the shape of the window 9. The window 9 may be of the same, or similar shape, to that of the outer periphery of the border layer 20.
The window 9 is a square through hole in the adhesive skin contact layer 20. The window 9 is located centrally relative to the periphery of the adhesive skin contact layer 20. Each side of the window 9 is arranged parallel to each of the corresponding sides of the adhesive skin contact layer 20. The window may have an area of 12,500mm$^2$. The skilled person will appreciate that the window 9 may be enlarged by any suitable means, for example by cutting the adhesive skin contact layer 20. The window 9 in the adhesive skin contact layer 20 enables the wound of a patient to contact, or be in fluid communication with, other layers of the wound dressing 1, for example the wound contact layer 4, when the wound dressing 1 is applied to the skin of a patient.
The adhesive skin contact layer 20 comprises an upper surface 20b and a lower surface 20a. The lower surface 20a contacts the dermal surface in use. In use, the lower surface 20a of the adhesive skin contact layer 20 is detachably adhered to a dermal surface (not shown) of a patient such that the wound site (not shown) is located within the window 9. As such, the adhesive skin contact layer 20 is not in direct contact with the wound site but does wholly surround it. Thus, the adhesive skin contact layer 20 is in contact with intact peri-wound skin only. The upper surface 20b may be outwardly facing away from the patient's body.

A removable cover or "release layer" (not shown) may be adhered to the lower surface 20a of the adhesive skin contact layer 20. The removable cover, which may comprise folded grip sections, is removable from the lower surface 20a of the adhesive skin contact layer 20. Thus, the removable cover protects the adhesive skin contact layer 20 when the removable cover is adhered to the adhesive skin contact layer 20, but when the removable cover is removed from the lower surface 20a of the adhesive skin contact layer 20 for use of the wound dressing 1, the lower surface 20a is exposed and able to releasably secure the wound dressing 1 to the skin of a patient.

The wound contact layer 4, the transmission layer 5 and the superabsorbent layer 6 form an absorbent structure arranged between the adhesive skin contact layer 20 and the backing layer 7.

The backing layer 7 may comprise a first surface 7a and a second surface 7b. In use, the first surface 7a may be inwardly facing toward the patient's body, and the second surface 7b may be outwardly facing away from the patient's body.

The absorbent structure 4, 5, 6 may comprise a first surface 4a and a second surface 6b. The first surface 4a of the absorbent structure 4, 5, 6 may be adjacent, and in contact with, the upper surface 20b of the adhesive skin contact layer 20. The peripheral edge of the second surface 6b of the absorbent structure 4, 5, 6 may be adjacent, and in contact with, the first surface 7a of the backing layer 7.

The wound contact layer 4 may comprise a first surface 4a and a second surface 4b. In use, the first surface 4a may be inwardly facing toward the patient's body, and the second surface 4b may be outwardly facing away from the patient's body. The first surface 4a of the wound contact layer 4 may contact the wound when the wound dressing 1 is adhered to the skin adjacent to the wound (i.e., when in use). The first surface 4a of the wound contact layer 4 is substantially aligned with the window 9 of the adhesive skin contact layer 20. As such, in use, the first surface 4a contacts the wound through the window 9. The second surface 4b of the wound contact layer 4 may be adjacent, and in contact with, a first surface 5a of the transmission layer 5 or a first surface 6a of the superabsorbent layer 6. In the described embodiment, the second surface 4b of the wound contact layer 4 is adjacent, and in contact with, the first surface 5a of the transmission layer 5.

The transmission layer 5 may comprise a first surface 5a and a second surface 5b. In use, the first surface 5a may be inwardly facing toward the patient's body, and the second surface 5b may be outwardly facing away from the patient's body. The first surface 5a of the transmission layer 5 may be adjacent, and in contact with, the second surface 4b of the wound contact layer 4 or a second surface 6b of the superabsorbent layer 6. The second surface 5b of the transmission layer 5 may be adjacent, and in contact with, a first surface 6a of the superabsorbent layer 6 or the first surface 7a of the backing layer 7. In the described embodiment, the first surface 5a of the transmission layer 5 is adjacent, and in contact with, the second surface 4b of the wound contact layer, and the second surface 5b of the transmission layer 5 is adjacent, and in contact with, the first surface 6a of the superabsorbent layer 6.

The superabsorbent layer 6 may comprise a first surface 6a and a second surface 6b. In use, the first surface 6a may be inwardly facing toward the patient's body, and the second surface 6b may be outwardly facing away from the patient's body. The first surface 6a of the superabsorbent layer 6 may be adjacent, and in contact with, the second surface 4b of the wound contact layer 4 or the second surface 5b of the transmission layer 5. The second surface 6b of the superabsorbent layer 6 may be adjacent, and in contact with, the first surface 5a of the transmission layer 5 or the first surface 7a of the backing layer 7. In the described embodiment, the first surface 6a of the superabsorbent layer 6 is adjacent, and in contact with, the second surface 5b of the transmission layer 5, and the second surface 6b of the superabsorbent layer 6 is adjacent, and in contact with, the first surface 7a of the backing layer 7.

The adhesive skin contact layer 20 may radially overlap the absorbent structure 4, 5, 6. The adhesive skin contact layer 20 may comprise an interior portion adjacent to and surrounding the window 9, and an exterior portion radially outwardly from the interior portion. The interior portion and the exterior portion of the first surface 20a of the adhesive skin contact layer 20 may contact and adhere to the skin of a patient in use. The interior portion of the second surface 20b of the adhesive skin contact layer 20 may be adjacent, and in contact with, a portion of the first surface 4a of the wound contact layer 4. The exterior portion of the second surface 20b of the adhesive skin contact layer 20 may be adjacent, and joined with, a peripheral portion of the first surface 7a of the backing layer 7. Beneficially, this helps to prevent wound exudate escaping from the confines of the wound dressing which would be unhygienic and cause discomfort for the user. Moreover, this also helps maintain a negative pressure at the wound site.

The exterior portion of the second surface 20b of the adhesive skin contact layer 20 may be joined to the peripheral portion of the first surface 7a of the backing layer 7 by heat lamination, adhesive, welding or stitching.

The exterior portion of the second surface 20b of the adhesive skin contact layer 20 may extend radially beyond the periphery of the absorbent structure by about 27.5 mm.

At least two of the wound contact layer 4, the transmission layer 5 and the superabsorbent layer 6 may be laminated together. In the described embodiment, each of the wound contact layer 4, the transmission layer 5 and the superabsorbent layer 6 are laminated together. Advantageously, this means that the absorbent structure of the present invention is manufactured without requiring any relatively rigid dressing and binding components. Thus, the present invention provides an absorbent structure which is flexible, therefore benefits user comfort, and which is cheaper to manufacture than absorbent structures and, therefore negative pressure wound dressings, of the prior art. Moreover, lamination does not require any complex equipment or manufacturing techniques. Thus, the present invention is simple to manufacture.

Each of the wound contact layer 4, the transmission layer 5 and the superabsorbent layer 6 may be laminated together by a scatter-coat adhesive. The scatter-coat adhesive may be a hot melt, scatter-coat adhesive. In the described embodiment, the scatter-coat adhesive is polycaprolactone and has the following properties:

- a melt flow index of between 5.2 g/10min and 11.3 g/10min, as tested with 2.16 kg, 1" PVC die at 160°C;

- a maximum water content of 0.35%;

- a mean molecular weight of 50,000;

- a melting point of 60°C; and

- a solubility parameter of 9.34 cal/cm3 to 9.43 cal/cm3.

The scatter-coat adhesive may comprise particles of which at least 98% have a particle size of 0.6mm.

The wound contact layer 4, the transmission layer 5 and the superabsorbent layer 6 may be laminated together by a laminator having the following processing properties:

- Heating zone temperature of 125°C;

- Cooling zone temperature of 25°C;

- Web tension (for each unwind reel) of 125 N;

- Crush roller pressure of 1000 N; and

- Web speed of 10 m/min.

The wound contact layer 4 may further comprise a central region 4c and a peripheral region 4d. The central region 4c may be substantially square and may be bordered on each side by the peripheral region 4d.

In the described embodiment, the first surface 4a, which contacts the wound in use, may comprise warp stitches (longitudinal to the plane of the wound contact layer 4) only, i.e., the first surface 4a does not comprise any weft stitches (transverse to the plane of the wound contact layer 4). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface 4a may have a pitch of 6.6mm and a stitch density of 7.5 stitches /cm.

The second surface 4b, which is laminated to the first surface 5a of the transmission layer 5, may comprise warp stitches and weft stitches. Each of the warp stitches and the weft stitches of the second surface 4b have a pitch of 6.6mm and a stitch density of 7.5 stitches /cm.

In other embodiments, the central region 4c of the first surface 4a may comprise warp stitches only and the peripheral region 4d of the first surface 4a may comprise warp and weft stitches.

The wound contact layer 4 may have a thickness of 3.0 mm.

The wound contact layer 4 may comprise two layers (layers not shown in Figures). Each layer may be substantially square and have a thickness of about 1.5 mm. Each layer may be substantially planar. Each layer may be formed of a plurality of gel-forming fibres which, in the present embodiment, are sodium carboxymethylcellulose fibres. The sodium carboxymethylcellulose fibres may have a degree of substitution of about 0.30 carboxymethyl groups per glucose unit, as measured by IR spectroscopy (as defined in WO00/01425). Each layer may have a basis weight of about 100 g/m$^2$. An antimicrobial agent comprising ionic silver may be dispersed upon the sodium carboxymethylcellulose fibres of each layer of the wound contact layer 4. The ionic silver may be present in the antimicrobial agent at a concentration of about 1.20 wt.%. The two layers may be stitchbonded together with lyocell yarns (produced under the trade name Tencel™ yarn) about the periphery of each layer.

The layers may be arranged such that the first surface 4a of the wound contact layer 4 is a first surface of the first layer, and the second surface 4b of the wound contact layer 4 is a second surface of the second layer. The second surface of the first layer and the first surface of the second layer may be arranged against one another. The two layers, therefore, form a wound contact layer having a total basis weight of 200 g/m$^2$.

The sodium carboxymethylcellulose fibres are hygroscopic fibres which upon the uptake of wound exudate become gelatinous. The sodium carboxymethylcellulose fibres may be of the type which retain their structural integrity on absorption of exudate.

The absence of weft stitches at the first surface 4a means that a greater amount of gel-forming fibres, as described above, are exposed at the first surface and, therefore, in contact with the wound. As such, the wound contact layer is higher gelling, i.e., has a significantly greater absorbency capacity and is more retentive, than gel-forming fibres of the prior art. As such, the wound contact layer 4 absorbs a greater amount of wound exudate. Thus, pooling of wound exudate at the wound site is prevented, or at least significantly reduced.

The sodium carboxymethylcellulose fibres are made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres have an absorbency of 2 grams 0.9% saline solution per gram of fibre (as measured by the free swell method).

The wound contact layer may further comprise an antibiofilm agent. The antibiofilm agent may comprise ethylenediaminetetra-acetic acid (EDTA). In other embodiments, the antibiofilm agent may comprise a salt of EDTA, for example a di-sodium or calcium di-sodium salt, or a combination of EDTA and one or more of its salts. The EDTA may be present in an amount of about 2.0 wt.%.

The antibiofilm agent may further comprise a quaternary cationic surfactant. In the present embodiment, the quaternary cationic surfactant is benzyl ammonium chloride. The benzyl ammonium chloride may be present in an amount of about 0.75 wt.%.

The antimicrobial agent may further comprise a buffering agent which, in the present embodiment, may be citric acid.

In the present embodiment, the wound contact layer 4 does not comprise any silicone, i.e., comprises 0 wt.% silicone.

The transmission layer 5 is substantially square, as is the wound contact layer 4 and the superabsorbent layer 6, and has a thickness of 1.8mm and is made of a polyurethane foam. The transmission layer 5 may have a density of between 26 Kg/M$^3$ and 28 Kg/M$^3$ and a tensile strength of 150 kPa. The transmission layer 5 has an elongation at break value of 300% and a cell count of between 17 and 26 /cm. Moreover, the transmission layer 5 has a 40% CLD hardness of between 2.5 and 4.5 kPa.

The superabsorbent layer 6 comprises an upper layer having the second surface 6b, and a lower layer having the first surface 6a. Between the upper layer and the lower layer, there is enclosed a first material comprising non-woven fibres, an absorbent material and a hot melt binder.

The hot melt binder may be present in an amount of 70 g/m$^2$. The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 6 may each be a cellulosic fabric layer. The upper fabric layer and the lower fabric layer may be joined around their entire periphery to enclose the first material.

The superabsorbent layer 6 may comprise a plurality of fenestrations (not shown). Each of the plurality of fenestrations may be openings in the form of slits in the superabsorbent layer 6. Each fenestration may have a length equal to 80% of the length of the superabsorbent layer 6. Each of the upper layer and the lower layer may comprise a plurality of fenestrations. The fenestrations may be arranged in a direction planar to a longitudinal axis and transverse to a longitudinal axis of the superabsorbent layer 6. Advantageously, this arrangement aids in managing a flow of exudate through the superabsorbent layer 6 of the wound dressing 1. The fenestrations encourage or enable wound exudate to travel laterally on the superabsorbent layer 6, as opposed to axially, and assist in negative pressure transmission through the superabsorbent layer 6.

The superabsorbent layer 6 may have a basis weight of 460 $g/m^2$, a thickness of 2.55 mm and a density of 0.255 $g/cm^3$. Moreover, the superabsorbent layer 6 may have a tensile strength (dry) of 45 N/50mm, an absorbent capacity of 25 g/g 10min, and an absorbency under compression capacity of 0.85 $g/cm^2$.

Each of the wound contact layer 4, transmission layer 5 and superabsorbent layer 6 are substantially planar.

In use, the adhesive skin contact layer 20 is adhered to the intact peri-wound skin around a wound. The wound is located within the window 9 of the adhesive skin contact layer 20. The wound dressing 1 is connected to a source of negative pressure before or after application of the dressing to the wound. Upon leaving the wound, wound exudate contacts the first surface 4a of the wound contact layer 4 and wicks upwardly, away from the wound site, toward the transmission layer 5. The high gelling ability of the wound contact layer 4 facilitates absorption and retention of the wound exudate. The antimicrobial agent comprising ionic silver provided an antimicrobial effect to the wound site. Lamination of the wound contact layer 4, transmission layer 5 and superabsorbent layer 6 means each of these layers form a consolidated, absorbent and retentive structure. The wound exudate wicks from the upper surface 4b of the wound contact layer 4 to the transmission layer 5. Lateral movement of the exudate occurs in the transmission layer 5 to spread the wound exudate across the surface area of the dressing. The exudate then wicks upwardly to the superabsorbent layer 6 where it is absorbed and retained prior to being transmitted out of the dressing 1 via vapour transmission through the backing film 7.

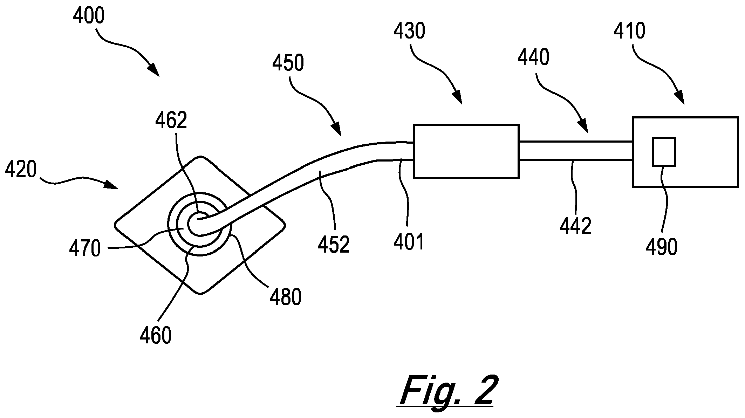

With reference to Figure 2, illustrated therein is a negative pressure wound exudate management system 400 according to certain embodiments. The negative pressure wound exudate management system 400 comprises a pump 410 for generating negative pressure, a wound dressing 420 (which may be the wound dressing 1 described above) for covering and protecting a wound (not shown), an inline filter 430, a first pressure tube 440 having a first interior lumen 442, a second pressure tube 450 (which may be the airway 8 described above) having a second interior lumen 452, and a flexible connector 460. The first pressure tube 440 is disposed between the pump 410 and the inline filter 430. The second pressure tube 450 is disposed between the inline filter 430 and the flexible connector 460. The first pressure tube 440 and/or the second pressure tube 450 may comprise a one-way valve (not shown) (i.e., in-line between the pump 410 and the wound dressing 420) to maintain a negative pressure within the wound dressing 420 when the pump 410 is disconnected from the first and/or second pressure tube 440, 450. The flexible connector 460 is disposed between the second pressure tube 450 and a backing layer of the wound dressing 420. The backing layer comprises an aperture (not shown) and the flexible connector 460 is sealed 480, for example by an adhesive or weld line, to the backing layer around the periphery of the aperture, such that the pump 410 and the wound dressing 420 are in fluid communication via the interior lumens 442, 452.

The following Assessment was performed to demonstrate the antimicrobial efficacy of the negative pressure wound dressing according to the invention.

**Assessment 1 - Wound contact layer comprising an antimicrobial agent comprising ionic silver**

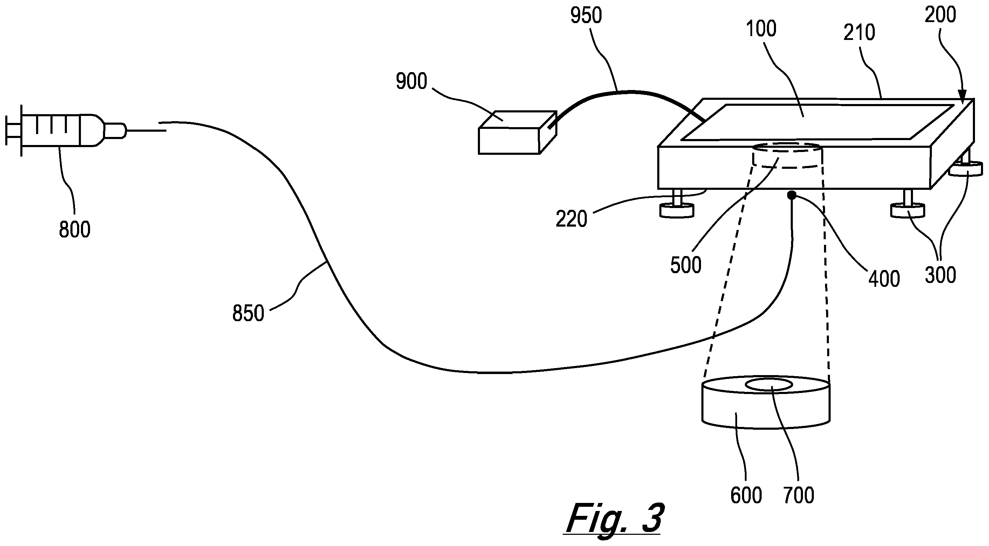

To demonstrate the antimicrobial efficacy of the negative pressure wound dressing according to the invention, an apparatus was arranged as schematically shown in Figure 3.

*Apparatus comprising the negative pressure wound dressing according to the invention*

The apparatus comprised a negative pressure wound dressing 100 which was identical to the wound dressing 1 described above and shown in Figure 1. In particular, the wound dressing 100 was a negative pressure wound dressing 1 comprising a wound contact layer 4 comprising two layers. Each layer was substantially square and had a thickness of about 1.5 mm. Each layer was substantially planar. Each layer was formed of a plurality of gel-forming fibres which were sodium carboxymethylcellulose fibres. The sodium carboxymethylcellulose fibres had a degree of substitution of about 0.30 carboxymethyl groups per glucose unit, as measured by IR spectroscopy (as defined in WO00/01425). Each layer had a basis weight of about 100 $g/m^2$. An antimicrobial agent comprising ionic silver was dispersed upon the sodium carboxymethylcellulose fibres of each layer of the wound contact layer 4. The ionic silver was present in the antimicrobial agent at a concentration of about 1.20 wt.%. The two layers were stitchbonded together with lyocell yarns (produced under the trade name Tencel™ yarn) about the periphery of each layer.

The wound contact layer further comprised an antibiofilm agent comprising a chelating agent. The chelating agent comprised ethylenediaminetetra-acetic acid (EDTA) in an amount of about 2.0 wt.%.

The antibiofilm agent further comprised a quaternary cationic surfactant. The quaternary cationic surfactant was benzyl ammonium chloride. The benzyl ammonium chloride was present in an amount of about 0.75 wt.%.

The antimicrobial agent further comprised a buffering agent which was citric acid.

The wound contact layer did not comprise any silicone, i.e., the wound contact layer comprised 0 wt.% silicone.

Other features of the wound dressing 100 were as described above and as shown in Figure 1.

The wound dressing 100 was attached, via its adhesive skin contact layer, to an upper surface 210 of a test rig 200. The test rig 200 comprised four feet 300 attached to its lower surface 220 to provide a steady and planar surface for the wound dressing 100. The lower surface 220 of the test rig 200 comprised a fluid inlet 400 to which was attached tubing 850 having a syringe 800 at the distal end of the tubing 850.

A pump 900 provided a source of negative pressure to the wound dressing 100 through an internal lumen of a conduit 950 which was attached to an airway (not shown) of the wound dressing 100.

Wound exudate was simulated using Simulated Wound Fluid (SWF) which is a fluid comprised of Maximum Recovery Diluent (MRD) and Foetal Bovine Serum (FBS) in equal quantities. The compositions of MRD and FBS are known in the art. The SWF was pumped from the syringe 800 to the fluid inlet 400 at a rate of 0.898 ml/hour.

A wound site was simulated using a 53mm deep well 500. The well 500 comprised an agar plate 600 colonised with a challenge microorganism. The agar plate 600 simulated a wound surface. Arranged at an approximate central location on the agar plate surface was a filter paper 700. In the Assessment, the filter paper 700 was the medium which was examined for bacterial growth.

*Comparative Apparatus*

A comparative apparatus was provided which comprised the features of the Apparatus described above but differing in that the wound contact layer of the wound dressing did not comprise an antimicrobial agent. As such, the wound contact layer of the wound dressing of the comparative apparatus did not comprise any ionic silver.

*Assessment*

The wound dressing 100 was pre-conditioned with SWF for 7 days. In this time, the well 500 was not inserted into the test rig 200 and so there was no challenge bacteria in contact with the wound dressing 100.

After pre-conditioning, the filter paper 700 was inoculated with 1 x $10^6$ CFU/10$\mu$l of a challenge microorganism, as specified below.

The well 500 was attached to the lower surface 220 of the test rig 200, below an approximate central portion of the wound dressing 100, such that the inoculated filter paper 700 was in contact with the wound contact layer of the wound dressing.

The apparatus was incubated and SWF pumped toward the wound dressing 100 to represent a moderately exuding wound, together with application of a negative pressure by activating the pump 900. The negative pressure at the simulated wound site was 116.7 kPa (125mmHg).

A total viable cell count of microorganism present on the filter paper 700 was taken at 0, 6, 24, 48 and 168 hours from incubation.

An accepted 'pass' criteria as established by regulatory agencies (for example the Food and Drug Administration (FDA) of the United States) is a 4-log reduction of the total viable cell count within 48 hours of incubation.

*Microorganisms*

Separate tests were carried out using different microorganisms: Pseudomonas aeruginosa NCTC 8506 (antibiotic resistant) (bacteria), Staphylococcus aureus ATCC BAA-1556 (MRSA) (bacteria), and Candida krusei NCPF 3876 (yeast).

Preparation of each agar plate 600:

- A working suspension of ~1 x $10^8$ CFU/ml was prepared in Maximum Recovery Diluent using representative colonies of the challenge microorganism,
- TSA + 3% AB (Bacteriological Agar) agar disc is placed on top of blotting paper into the test rig 200,
- A 25mm Whatman filter was placed on top of the agar disc and inoculated with 10$\mu$l of the working suspension (~1 x $10^6$ CFU/10$\mu$l).

*Analysis*

Example 1

In this Example, the filter paper of both the apparatus comprising a wound contact layer according to the invention (wound contact layer comprising an antimicrobial agent comprising 1.20 wt.% ionic silver) and the comparative apparatus (wound contact layer not comprising an antimicrobial agent) were inoculated with Pseudomonas aeruginosa NCTC 8506 (antibiotic resistant) (*P. Aeruginosa* NCTC 8506) bacteria.

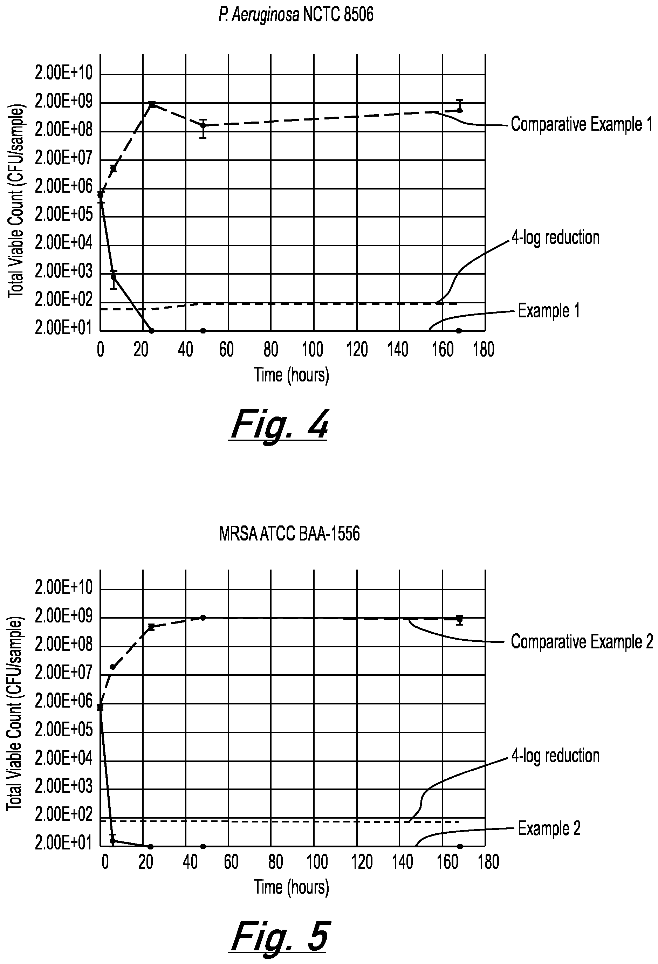

As shown in Figure 4, the wound dressing of the invention caused a sharp decrease in the total viable cell count of bacteria after inoculation. A 4-log reduction of the total viable cell count of *P. Aeruginosa* NCTC 8506 was achieved within 24 hours of inoculation. The 4-log reduction of the total viable cell count of *P. Aeruginosa* NCTC 8506 was maintained from 24 hours after incubation to completion of the 7-day (168 hour) test.

Comparatively, Comparative Example 1, which did not comprise an antimicrobial agent, allowed for around a 3-log increase in total viable cell count of *P. Aeruginosa* NCTC 8506 bacteria within 24 hours of incubation. A 4-log reduction of the total viable cell count 48 hours after incubation was not achieved by the Comparative Example.

Example 2

In this Example, the filter paper of both the apparatus comprising a wound contact layer according to the invention (wound contact layer comprising an antimicrobial agent comprising 1.20 wt.% ionic silver) and the comparative apparatus (wound contact layer not comprising an antimicrobial agent) were inoculated with Staphylococcus aureus ATCC BAA-1556 (MRSA) (MRSA ATCC BAA-1556).

As shown in Figure 5, the wound dressing of the invention caused a sharp decrease in the total viable cell count of the bacteria after incubation. A 4-log reduction of the total viable cell count of MRSA ATCC BAA-1556 was achieved within 6 hours of incubation. The 4-log reduction of the total viable cell count of MRSA ATCC BAA-1556 was maintained from 6 hours after incubation to completion of the 7-day (168 hour) test.

Comparatively, Comparative Example 2, which did not comprise an antimicrobial agent, allowed for around a 3-log increase in total viable cell count of MRSA ATCC BAA-1556 bacteria within 48 hours of incubation. A 4-log reduction of the total viable cell count 48 hours after incubation was not achieved by the Comparative Example.

Notably, Example 2 shows that a negative pressure wound dressing according to the invention has greater efficacy in eliminating MRSA ATCC BAA-1556 compared to *P. Aeruginosa* NCTC 8506 bacteria as used in Example 1.

Example 3

In this Example, the filter paper of both the apparatus comprising a wound contact layer according to the invention (wound contact layer comprising an antimicrobial agent comprising 1.20 wt.% ionic silver) and the comparative apparatus (wound contact layer not comprising an antimicrobial agent) were inoculated with Candida krusei NCPF 3876 (C. *krusei* NCPF 3876) yeast.

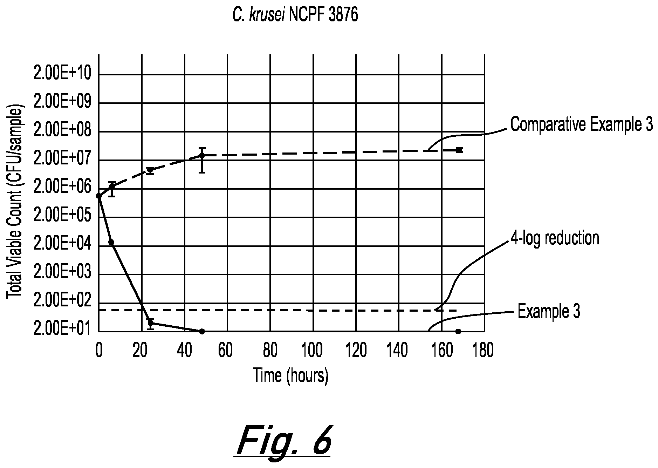

As shown in Figure 6, the wound dressing of the invention caused a decrease in the total viable cell count of the yeast after incubation. A 4-log reduction of the total viable cell count of *C. krusei* NCPF 3876 was achieved within 24 hours of incubation. The 4-log reduction of the total viable cell count of *C. krusei* NCPF 3876 was maintained from 24 hours after incubation to completion of the 7-day (168 hour) test.

Comparatively, Comparative Example 3, which did not comprise an antimicrobial agent, allowed for an increase in total viable cell count of *C. krusei* NCPF 3876 yeast up to around 48 hours from incubation. A 4-log reduction of the total viable cell count 48 hours after incubation was not achieved by Comparative Example 3.

*Conclusion*

It has been found that, for each of the three types of microorganisms tested, a negative pressure wound dressing according to the invention provides for a 4-log reduction of the total viable cell count of each bacteria within 48 hours of incubation.

The advantage of the present invention compared to a negative pressure wound dressing having a wound contact layer which does not comprise an antimicrobial agent comprising ionic silver is clearly displayed in Examples 1-3.

The following Assessment was performed to demonstrate the antimicrobial activity of a negative pressure wound dressing according to the invention, against bacteria, yeast and moulds.

**Assessment 2** - **In vitro direct inoculation simulated wound fluid model**

An in vitro Direct Inoculation Simulated Wound Fluid Model (based on an adapted AATCC TM100 - Antimicrobial Textile Test) has been used to evaluate the antimicrobial activity of a negative pressure wound dressing according to the invention against bacteria, yeast and moulds.

Overview

As described below, multiple wound contact layers as comprised in the negative pressure wound dressing of the invention were prepared, together with multiple samples of a wound contact layer which formed an experimental control, were pre-conditioned to mimic clinical conditions and each challenged with a bacteria, yeast or mould.

Method

*Microorganisms*

Separate tests were carried out using different microorganisms: vancomycin resistant Enterococcus faecalis NCTC 12201, Methicillin Resistant Staphylococcus aureus ATCC® BAA-1556™ (MRSA), Staphylococcus pyogenes NCTC 10872, Acinetobacter baumannii NCTC 13421, ESBL producing Klebsiella pneumoniae NCTC 13465, antibiotic resistant Pseudomonas aeruginosa NCTC 8506, Aspergillus brasiliensis NCPF® 2275 and Candida krusei NCPF® 3876.

*Sample preparation*

4cm x 4cm samples of a negative pressure wound dressing according to the invention were prepared. In each sample, the wound contact layer comprised two layers. The two layers were stitchbonded together with lyocell yarns (produced under the trade name Tencel™ yarn) about the periphery of each layer. Each layer was substantially square and had a thickness of about 1.5 mm. Each layer was substantially planar. Each layer was formed of a plurality of gel-forming fibres which were sodium carboxymethylcellulose fibres. The sodium carboxymethylcellulose fibres had a degree of substitution of about 0.30 carboxymethyl groups per glucose unit, as measured by IR spectroscopy (as defined in WO00/01425). Each layer had a basis weight of about 100 g/m2. An antimicrobial agent comprising ionic silver was dispersed upon the sodium carboxymethylcellulose fibres of each sample of wound contact layer. The ionic silver was present in the antimicrobial agent at a concentration of about 1.20 wt.%. The wound contact layer did not comprise any silicone, i.e., the wound contact layer comprised 0 wt.% silicone.

A further sample of a negative pressure wound dressing was prepared as described above, but with the exception that the wound contact layer of this sample did not comprise ionic silver. This sample formed the experimental control.

Each sample was transferred into a sterile container. Each sample (n=3) was then hydrated with 11.99ml Simulated Wound Fluid (SWF) (50:50 Maximum Recovery Diluent (MRD) : fetal bovine serum) and incubated at $35\pm3°C$ for 168 hours.

A working suspension of ~1 x $10^8$ cfu/ml was prepared in MRD using representative colonies of the challenge organism. After 168 hours incubation (pre-conditioning), the samples were inoculated with ~1 x $10^6$ cfu and re-incubated until their appropriate timepoints (0, 24, 48, 72, 96 and 168 hours).

At each timepoint, the appropriate samples were transferred into stomacher bags containing 40ml DENB and homogenised for 4 minutes on "high" within a laboratory stomacher. Total Viable Counts (TVCs) were performed on the resultant suspensions. The samples were incubated at $35\pm3°C$ (or 20-25°C for A. brasiliensis NCPF 2275 and C. krusei NCPF 3876) for at least 48 hours.

To determine antimicrobial activity, log reductions were calculated as follows:

Log reduction = $Log_{10}$ of control initial challenge level - $Log_{10}$ of count at specified timepoint

An accepted 'pass' criteria as established by regulatory agencies (for example the Food and Drug Administration (FDA) of the United States) is a 4-log reduction of the total viable cell count within 48 hours of incubation for bacteria, and within 168 hours (7 days) for yeast and mould.

Analysis

*Example 4*

**In** this Example, the negative pressure wound dressing of the invention (wound contact layer comprising an antimicrobial agent comprising 1.20 wt.% ionic silver) (Example 4) and the comparative Example (experimental control which does not comprise ionic silver) were inoculated with vancomycin resistant Enterococcus faecalis NCTC 12201.

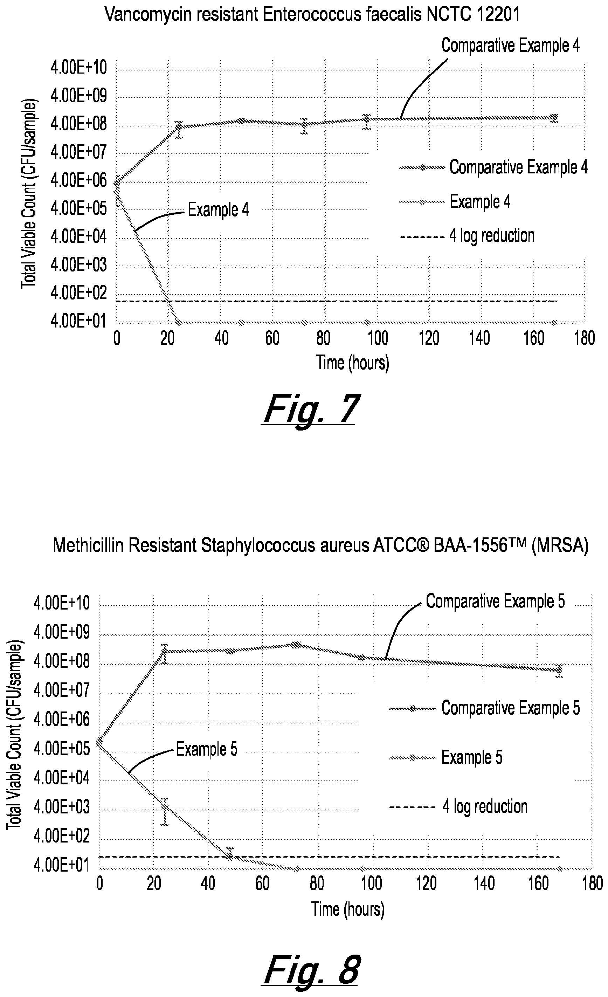

As shown in Figure 7, the negative pressure wound dressing of the invention caused a sharp decrease in the total viable cell count of vancomycin resistant Enterococcus faecalis NCTC 12201 within 24 hours. The 4-log reduction of the total viable cell count of vancomycin resistant Enterococcus faecalis NCTC 12201 was maintained from 24 hours after incubation to completion of the 7-day (168 hour) test.

Comparatively, Comparative Example 4 (the experimental control), which did not comprise ionic silver, allowed for a 2-log increase in total viable cell count of vancomycin resistant Enterococcus faecalis NCTC 12201 within 24 hours of incubation. A 4-log reduction of the total viable cell count 48 hours after incubation was not achieved by the Comparative Example.

*Example 5*

In this Example, the negative pressure wound dressing of the invention (wound contact layer comprising an antimicrobial agent comprising 1.20 wt.% ionic silver) (Example 5) and the comparative Example (experimental control which does not comprise ionic silver) were inoculated with Methicillin Resistant Staphylococcus aureus ATCC® BAA-1556™ (MRSA).

As shown in Figure 8, Example 5 caused a decrease in the total viable cell count of Methicillin Resistant Staphylococcus aureus ATCC® BAA-1556™ (MRSA) with a 4-log reduction being achieved at around 48 hours from incubation. The total viable cell count of Methicillin Resistant Staphylococcus aureus ATCC® BAA-1556™ (MRSA) was further reduced with increasing time, and maintained at least a 4-log reduction of the total viable cell count to completion of the 7-day (168 hour) test.

Comparatively, Comparative Example 5 (the experimental control), which did not comprise ionic silver, allowed for a 3-log increase in total viable cell count of Methicillin Resistant Staphylococcus aureus ATCC® BAA-1556™ (MRSA) within 24 hours of incubation. A 4-log reduction of the total viable cell count 48 hours after incubation was not achieved by Comparative Example 5.

*Example 6*

In this Example, the negative pressure wound dressing of the invention (wound contact layer comprising an antimicrobial agent comprising 1.20 wt.% ionic silver) (Example 6) and the comparative Example (experimental control which does not comprise ionic silver) were inoculated with Staphylococcus pyogenes NCTC 10872.

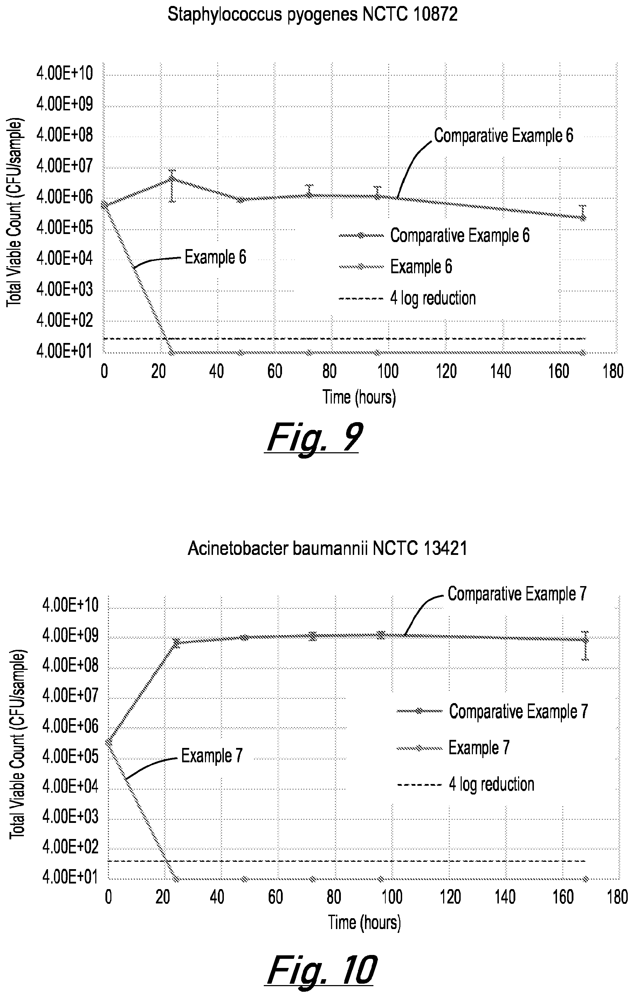

As shown in Figure 9, Example 6 caused a sharp decrease in the total viable cell count of Staphylococcus pyogenes NCTC 10872 within 24 hours. The 4-log reduction of the total viable cell count of Staphylococcus pyogenes NCTC 10872 was maintained from 24 hours after incubation to completion of the 7-day (168 hour) test.

Comparatively, Comparative Example 6 (the experimental control), which did not comprise ionic silver, allowed for an increase in the total viable cell count of Staphylococcus pyogenes NCTC 10872 within 24 hours of incubation. The total viable cell count of Comparative Example 6 did decrease to incubation levels at 48 hours and this was largely maintained for the duration of the test, with a small reduction in the total viable cell count at 168 hours. A 4-log reduction of the total viable cell count 48 hours after incubation was not achieved by the Comparative Example.

*Example 7*

**In** this Example, the negative pressure wound dressing of the invention (wound contact layer comprising an antimicrobial agent comprising 1.20 wt.% ionic silver) (Example 7) and the comparative Example (experimental control which does not comprise ionic silver) were inoculated with Acinetobacter baumannii NCTC 13421.

As shown in Figure 10, Example 7 caused a sharp decrease in the total viable cell count of Acinetobacter baumannii NCTC 13421 within 24 hours. The 4-log reduction of the total viable cell count of Acinetobacter baumannii NCTC 13421 was maintained from 24 hours after incubation to completion of the 7-day (168 hour) test.

Comparatively, Comparative Example 7 (the experimental control), which did not comprise ionic silver, allowed for a 3-log increase in total viable cell count of Acinetobacter baumannii NCTC 13421 within 24 hours of incubation. A 4-log reduction of the total viable cell count 48 hours after incubation was not achieved by Comparative Example 7.

*Example 8*

In this Example, the negative pressure wound dressing of the invention (wound contact layer comprising an antimicrobial agent comprising 1.20 wt.% ionic silver) (Example 8) and the comparative Example (experimental control which does not comprise ionic silver) were inoculated with ESBL producing Klebsiella pneumoniae NCTC 13465.

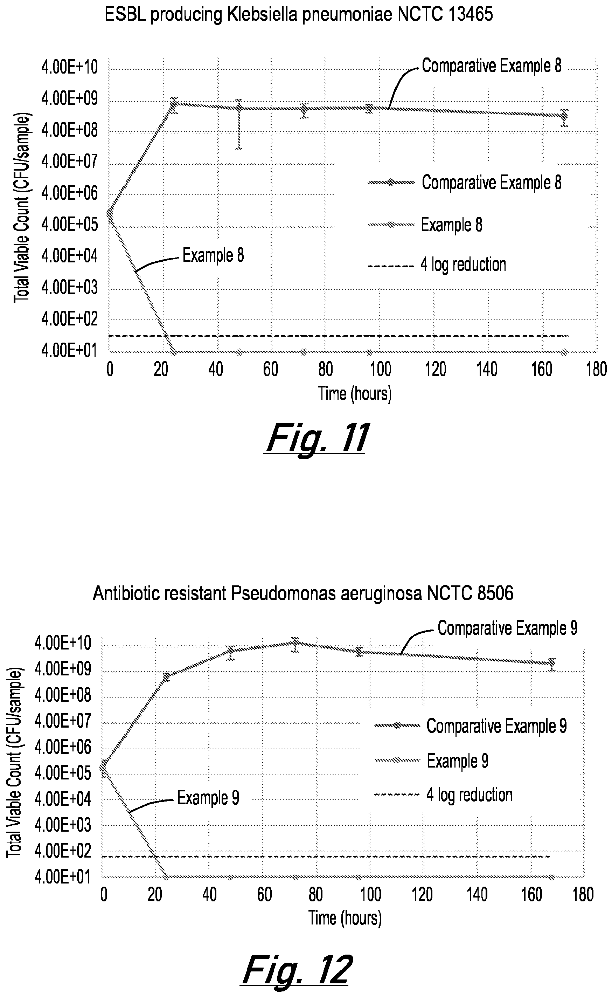

As shown in Figure 11, Example 8 caused a sharp decrease in the total viable cell count of ESBL producing Klebsiella pneumoniae NCTC 13465 within 24 hours. The 4-log reduction of the total viable cell count of ESBL producing Klebsiella pneumoniae NCTC 13465 was maintained from 24 hours after incubation to completion of the 7-day (168 hour) test.

Comparatively, Comparative Example 8 (the experimental control), which did not comprise ionic silver, allowed for a 3-log increase in total viable cell count of ESBL producing Klebsiella pneumoniae NCTC 13465 within 24 hours of incubation. A 4-log reduction of the total viable cell count 48 hours after incubation was not achieved by Comparative Example 8.

*Example 9*

In this Example, the negative pressure wound dressing of the invention (wound contact layer comprising an antimicrobial agent comprising 1.20 wt.% ionic silver) (Example 9) and the comparative Example (experimental control which does not comprise ionic silver) were inoculated with antibiotic resistant Pseudomonas aeruginosa NCTC 8506.

As shown in Figure 12, Example 9 caused a sharp decrease in the total viable cell count of antibiotic resistant Pseudomonas aeruginosa NCTC 8506 within 24 hours. The 4-log reduction of the total viable cell count of antibiotic resistant Pseudomonas aeruginosa NCTC 8506 was maintained from 24 hours after incubation to completion of the 7-day (168 hour) test.

Comparatively, Comparative Example 9 (the experimental control), which did not comprise ionic silver, allowed for a 3-log increase in total viable cell count of antibiotic resistant Pseudomonas aeruginosa NCTC 8506 within 24 hours of incubation, and a 4-log increase in total viable cell count of antibiotic resistant Pseudomonas aeruginosa NCTC 8506 after 48 hours. A 4-log reduction of the total viable cell count 48 hours after incubation was not achieved by Comparative Example 9.

*Example 10*

In this Example, the negative pressure wound dressing of the invention (wound contact layer comprising an antimicrobial agent comprising 1.20 wt.% ionic silver) (Example 9) and the comparative Example (experimental control which does not comprise ionic silver) were inoculated with Aspergillus brasiliensis NCPF® 2275.

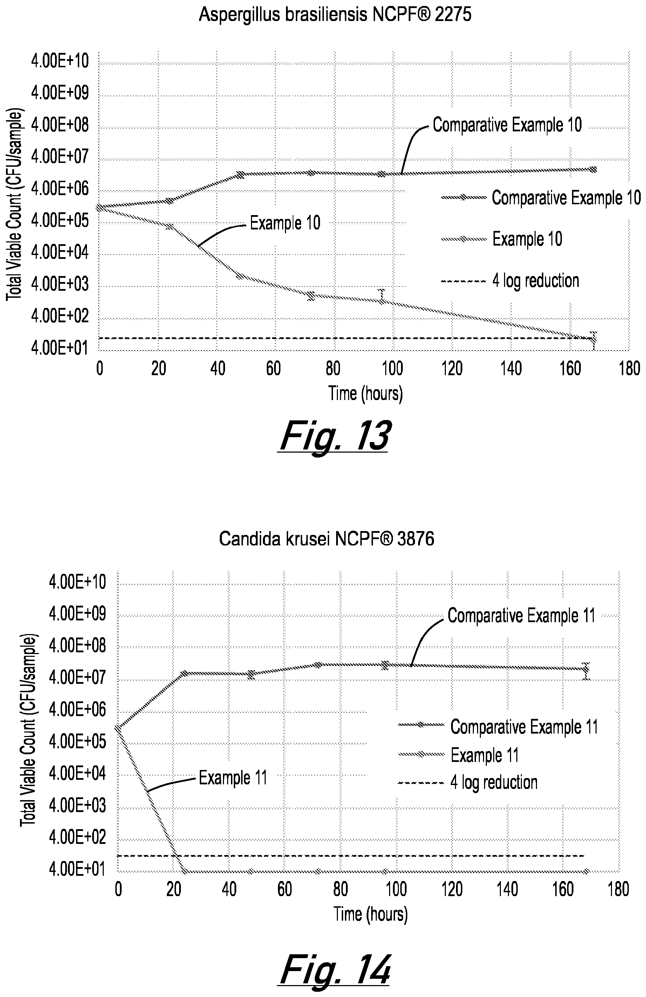

As shown in Figure 13, Example 10 caused a decrease in the total viable cell count of Aspergillus brasiliensis NCPF® 2275 within 24 hours, with a further decrease in the total viable cell count being observed at each measured time point. The 4-log reduction of the total viable cell count of Aspergillus brasiliensis NCPF® 2275 was achieved by 168 hours after incubation.

Comparatively, Comparative Example 10 (the experimental control), which did not comprise ionic silver, allowed for a 1-log increase in total viable cell count of Aspergillus brasiliensis NCPF® 2275 within 48 hours of incubation, and this was maintained to completion of the 7-day (168 hour) test. A 4-log reduction of the total viable cell count 48 hours after incubation was not achieved by Comparative Example 10.

*Example 11*

In this Example, the negative pressure wound dressing of the invention (wound contact layer comprising an antimicrobial agent comprising 1.20 wt.% ionic silver) (Example 9) and the comparative Example (experimental control which does not comprise ionic silver) were inoculated with Candida krusei NCPF® 3876.

As shown in Figure 14, Example 11 caused a sharp decrease in the total viable cell count of Candida krusei NCPF® 3876 within 24 hours. The 4-log reduction of the total viable cell count of Candida krusei NCPF® 3876 was maintained from 24 hours after incubation to completion of the 7-day (168 hour) test.

Comparatively, Comparative Example 11 (the experimental control), which did not comprise ionic silver, allowed for an increase in the total viable cell count of Candida krusei NCPF® 3876 within 24 hours, with a 2-log increase being achieved within 72 hours of incubation, and this was largely maintained to completion of the 7-day (168 hour) test. A 4-log reduction of the total viable cell count 48 hours after incubation was not achieved by Comparative Example 11.

Moreover, the log reduction in antimicrobial activity of a negative pressure wound dressing of the invention against challenge organisms during the 168 hour testing period is shown in Table 1.

Table 1

| | *E. faecalis* NCTC 12201 | *MRSA* ATCC BAA-1556 | *S. pyogenes* NCTC 10872 | *A. baumannii* NCTC 13421 | *K. pneumoniae* NCTC 13465 | *P. aeruginosa* NCTC 8506 | *A. brasiliensis* NCPF® 2275 | *C. krusei* NCPF® 3876 |
|---|---|---|---|---|---|---|---|---|
| 24 hours | 4.78 | 2.27 | 4.43 | 4.60 | 4.53 | 4.63 | 0.50 | 4.52 |

(continued)

|  | E. faecalis NCTC 12201 | MRSA ATCC BAA-1556 | S. pyogenes NCTC 10872 | A. baumannii NCTC 13421 | K. pneumoniae NCTC 13465 | P. aeruginosa NCTC 8506 | A. brasiliensis NCPF® 2275 | C. krusei NCPF® 3876 |
|---|---|---|---|---|---|---|---|---|
| 48 hours | 4.78 | 4.02 | 4.43 | 4.60 | 4.53 | 4.63 | 2.07 | 4.52 |
| 72 hours | 4.78 | 4.42 | 4.43 | 4.60 | 4.53 | 4.63 | 2.66 | 4.52 |
| 96 hours | 4.78 | 4.42 | 4.43 | 4.60 | 4.53 | 4.63 | 2.85 | 4.52 |
| 168 hours | 4.78 | 4.42 | 4.43 | 4.60 | 4.53 | 4.63 | 4.06 | 4.52 |

It is noted that in each of Figures 7 to 14, the error bars represnt standard deviation for n=3 at each timepoint.

*Conclusion*

Examples 4 to 9 and 11 demonstrate that, within 48 hours, the antimicrobial activity of a negative pressure wound dressing of the invention decreases the microbial population (total viable cell count) of vancomycin resistant E. *faecalis* NCTC 12201, MRSA ATCC® BAA-1556™, S. *pyogenes* NCTC 10872, A. *baumannii* NCTC 13421, ESBL producing K. *pneumoniae* NCTC 13465, antibiotic resistant P. *aeruginosa* NCTC 8506 and C. *krusei* NCPF® 3876 by more than 4-log reduction (compared to initial challenge level) which was then maintained throughout the remainder of the testing period.
Example 10 demonstrates that the antimicrobial activity of a negative pressure wound dressing of the invention decreases the total viable cell count of A. *brasiliensis* NCPF® 2275 by more than 4-log reduction (compared to initial challenge level).
The log reduction values for all challenge organisms at each timepoint of the testing period are shown within Table 1, which also demonstrates that all the bacteria cell number reached a 4 log reduction within 48 hours and yeast and mould cell number within 168 hours.
The following Assessment was performed to demonstrate the antibiofilm efficacy of a negative pressure wound dressing according to the invention, against different microorganisms.

**Assessment 3 - Antibiofilm efficacy**

An in vitro assessment was used to evaluate the antibiofilm efficacy of a negative pressure wound dressing according to the invention against single species biofilms of three clinically relevant microorganisms. More specifically, the ability of dressings to reduce biofilm was evaluated using a CDC Biofilm Reactor® model using polycarbonate disc coupons.

Overview

The in vitro assessment tests a negative pressure wound dressing according to the invention against biofilms grown on a polycarbonate coupon within a CDC Biofilm Reactor®. The CDC Biofilm Reactor® utilises continuous agitation to encourage the growth and microorganism cell attachment to a polycarbonate coupon. The polycarbonate coupon is rinsed in saline to remove planktonic cells and enveloped between sections of hydrated wound dressing according to the invention. Following required dressing exposure time, the coupon is removed and transferred to neutralising broth to be sonicated and vortexed, and a standard plate count performed to recover any remaining attached organism to the coupon. In addition, a total viable count was performed on the dressing. Testing was performed over 7 days (168 hours).

Method

*Microorganisms*

Separate tests (n=3) were carried out using different microorganisms: Staphylococcus aureus NCIMB 9518, Pseudomonas aeruginosa NCIMB 8626 or Candida krusei NCPF® 3876.

*Sample preparation*

Using 1ml of an overnight culture of *P. aeruginosa* NCIMB 8626 and S. *aureus* NCIMB 9518 and a 48-hour culture of C. *krusei* NCPF® 3876 adjusted to ~$10^8$ CFU/ml, the bioreactor was inoculated and made up to a final volume of 300ml nutrient medium (tryptone soy broth for bacteria and sabouraud dextrose liquid medium for C. *krusei*). The biofilm reactor was incubated at 35°C ($\pm$3°C) for 72 hours whilst shaking at 50 rpm. Each CDC bioreactor contains 24 polycarbonate coupons in 8 rods.

After 72 hours incubation, the rods containing the polycarbonate coupons within the CDC Biofilm Reactor® were removed and rinsed in saline to remove planktonic cells. The rinsed coupons were transferred to a 6-well cell culture plate and placed on the centre of a 44mm diameter section of a negative pressure wound dressing according to the invention such that the wound contact layer of the dressing was in contact with the coupons comprising the biofilm. The wound dressing was hydrated with 8ml Simulated Wound Fluid (SWF). In each section of wound dressing, the wound contact layer comprised two layers. The two layers were stitchbonded together with lyocell yarns (produced under the trade name Tencel™ yarn) about the periphery of each layer. Each layer was substantially square and had a thickness of about 1.5 mm. Each layer was substantially planar. Each layer was formed of a plurality of gel-forming fibres which were sodium carboxymethylcellulose fibres. The sodium carboxymethylcellulose fibres had a degree of substitution of about 0.30 carboxymethyl groups per glucose unit, as measured by IR spectroscopy (as defined in WO00/01425). Each layer had a basis weight of about 100 g/m2. An antimicrobial agent comprising ionic silver was dispersed upon the sodium carboxymethylcellulose fibres of each sample of wound contact layer. The ionic silver was present in the antimicrobial agent at a concentration of about 1.20 wt.%. The wound contact layer did not comprise any silicone, i.e., the wound contact layer comprised 0 wt.% silicone.

The wound dressing as described above was topped with a 35mm diameter section of a negative pressure wound dressing according to the invention such that the wound contact layer of this wound dressing was in contact with the coupons comprising the biofilm, and such that the coupons are sandwich by the two sections of wound dressing, specifically by the respective wound contact layer of each section of wound dressing. The 35mm diameter section of wound dressing was hydrated with 5ml SWF. In this section of wound dressing having a diameter of 35mm, the wound contact layer comprised two layers. The two layers were stitchbonded together with lyocell yarns (produced under the trade name Tencel™ yarn) about the periphery of each layer. Each layer was substantially square and had a thickness of about 1.5 mm. Each layer was substantially planar. Each layer was formed of a plurality of gel-forming fibres which were sodium carboxymethylcellulose fibres. The sodium carboxymethylcellulose fibres had a degree of substitution of about 0.30 carboxymethyl groups per glucose unit, as measured by IR spectroscopy (as defined in WO00/01425). Each layer had a basis weight of about 100 g/m2. An antimicrobial agent comprising ionic silver was dispersed upon the sodium carboxymethylcellulose fibres of each sample of wound contact layer. The ionic silver was present in the antimicrobial agent at a concentration of about 1.20 wt.%. The wound contact layer did not comprise any silicone, i.e., the wound contact layer comprised 0 wt.% silicone.

A sterile 20g weight was placed on top of the dressings. The lid of the 6-well cell culture plate was affixed to the culture plate as appropriate, and incubated at 35$\pm$3°C for 168 hours.

Further sections of a negative pressure wound dressing were prepared and arranged with polycarbonate coupons as described above, but with the exception that these sections of wound dressing did not comprise ionic silver. This formed the experimental control.

At 0 hours, total viable cell counts were performed on the polycarbonate coupon. After 48 and 168 hours, total viable cell counts were performed on the polycarbonate coupon and the dressings. The polycarbonate coupon was transferred to 10ml DENB in a sterile universal, sonicated for 5 mins and vortexed on 'high' (950 rpm) for 5 minutes. The dressing samples were transferred to 40ml DENB and homogenised on 'high' for 4 minutes. Total viable cell counts were performed on the resultant suspension by performing a serial dilution in DENB and plating aliquots onto tryptone soya agar (TSA) and sabouraud dextrose agar (SDA) plates. All plates were incubated at 35$\pm$3°C (or 20-25°C for *C. krusei* NCPF 3876) for at least 48 hours.

To determine antibiofilm activity the log reduction was calculated as follows:

Log reduction = $\text{Log}_{10}$ of control initial challenge level - $\text{Log}_{10}$ of count at specified timepoint

Analysis

*Example 12*

In this Example, the bioreactor was inoculated with Candida krusei NCPF® 3876, as described above, and tested with a negative pressure wound dressing of the invention (wound contact layer comprising an antimicrobial agent comprising 1.20 wt.% ionic silver) (Example 12) and a comparative Example (experimental control which does not comprise ionic silver).

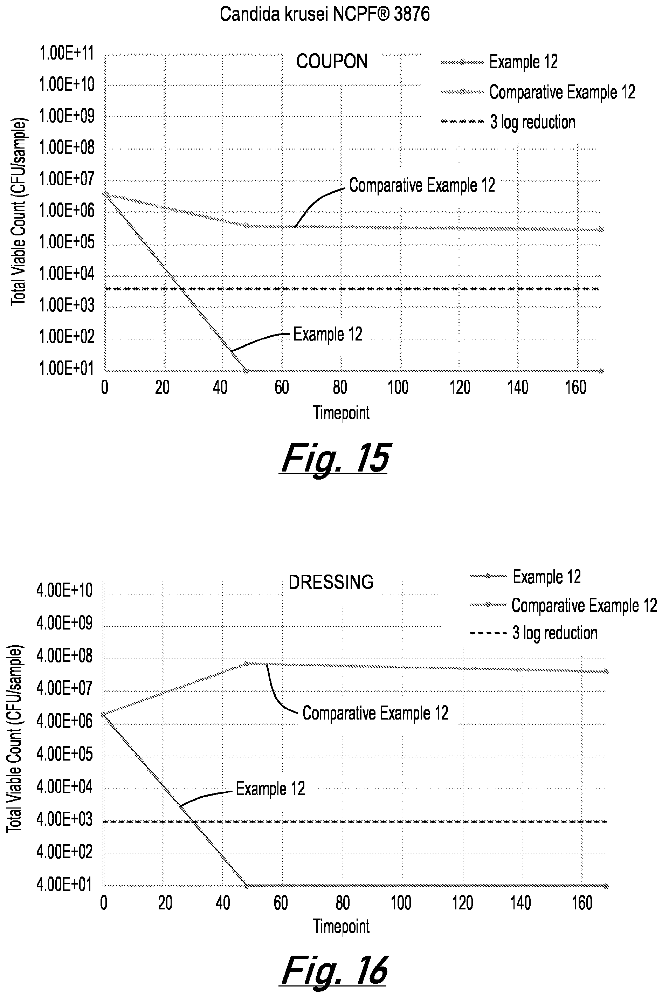

As shown in Figures 15 and 16, respectively, the polycarbonate coupon and the negative pressure wound dressing of the invention caused a greater than 3-log reduction in the total viable cell count of Candida krusei NCPF® 3876 within 48 hours. The greater than 3-log reduction of the total viable cell count of Candida krusei NCPF® 3876 was maintained from 48 hours after incubation to completion of the 7-day (168 hour) test.

Comparatively, Comparative Example 12 (the experimental control), which did not comprise ionic silver, allowed for an approximate 1-log decrease in the total viable cell count of Candida krusei NCPF® 3876 on the polycarbonate coupon within 48 hours of incubation, and a greater than 1-log increase in the total viable cell count of Candida krusei NCPF® 3876 on the dressing (of the comparative example) within 48 hours of incubation. A 3-log reduction of the total viable cell count 48 hours after incubation was not achieved by the Comparative Example.

*Example 13*

In this Example, the bioreactor was inoculated with Staphylococcus aureus NCIMB 9518, as described above, and tested with a negative pressure wound dressing of the invention (wound contact layer comprising an antimicrobial agent comprising 1.20 wt.% ionic silver) (Example 13) and a comparative Example (experimental control which does not comprise ionic silver).

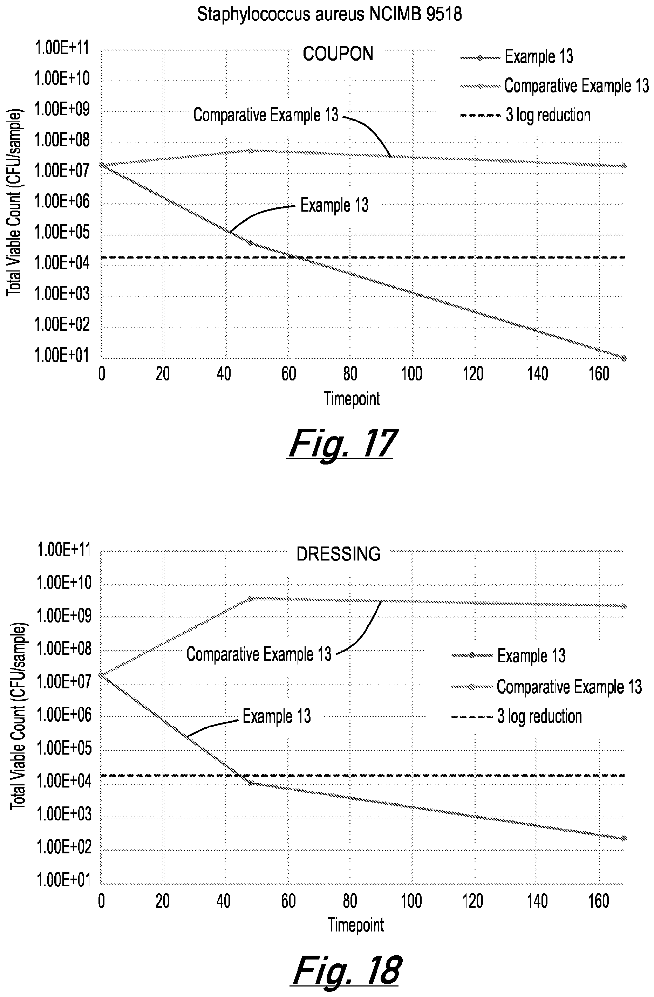

As shown in Figures 17 and 18, respectively, the polycarbonate coupon and the negative pressure wound dressing of the invention caused a greater than 3-log reduction in the total viable cell count of Staphylococcus aureus NCIMB 9518 within 168 hours and, in particular, within 48 hours with respect to the dressing test. The greater than 3-log reduction of the total viable cell count of Staphylococcus aureus NCIMB 9518 in respect of the dressing test was maintained from 48 hours after incubation to completion of the 7-day (168 hour) test.

Comparatively, Comparative Example 13 (the experimental control), which did not comprise ionic silver, allowed for an increase in the total viable cell count of Staphylococcus aureus NCIMB 9518 on the polycarbonate coupon within 48 hours of incubation, and 2-log increase in the total viable cell count of Staphylococcus aureus NCIMB 9518 on the dressing (of the comparative example) within 48 hours of incubation. A 3-log reduction of the total viable cell count 48 hours after incubation was not achieved by the Comparative Example.

*Example 14*

In this Example, the bioreactor was inoculated with Pseudomonas aeruginosa NCIMB 8626, as described above, and tested with a negative pressure wound dressing of the invention (wound contact layer comprising an antimicrobial agent comprising 1.20 wt.% ionic silver) (Example 14) and a comparative Example (experimental control which does not comprise ionic silver).

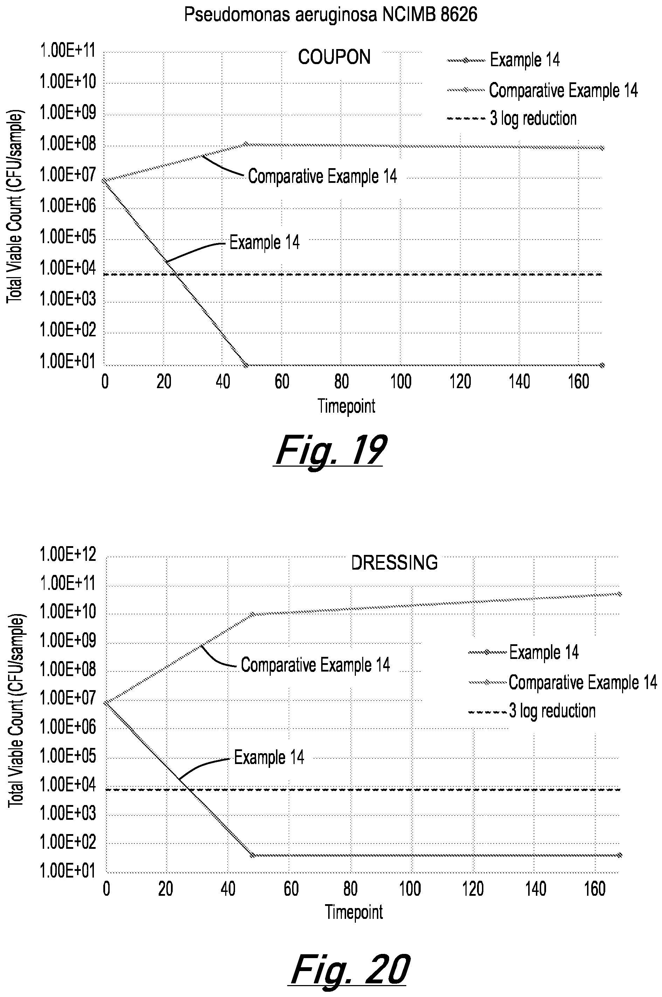

As shown in Figures 19 and 20, respectively, the polycarbonate coupon and the negative pressure wound dressing of the invention caused a greater than 3-log reduction in the total viable cell count of Pseudomonas aeruginosa NCIMB 8626 within 48 hours. The greater than 3-log reduction of the total viable cell count of Pseudomonas aeruginosa NCIMB 8626 was maintained from 48 hours after incubation to completion of the 7-day (168 hour) test.

Comparatively, Comparative Example 14 (the experimental control), which did not comprise ionic silver, allowed for an increase in the total viable cell count of Pseudomonas aeruginosa NCIMB 8626 on the polycarbonate coupon within 48 hours of incubation, and 3-log increase in the total viable cell count of Pseudomonas aeruginosa NCIMB 8626 on the dressing (of the comparative example) within 48 hours of incubation. A 3-log reduction of the total viable cell count 48 hours after incubation was not achieved by the Comparative Example.

*Conclusion*

Examples 12 to 14 demonstrate that, within 48 hours, a greater than 3-log reduction in the total viable cell count was observed for Candida krusei NCPF® 3876, Staphylococcus aureus NCIMB 9518 and Pseudomonas aeruginosa NCIMB 8626 when on each of the respective tested wound dressings.

Moreover, Examples 12 and 14 demonstrate that, within 48 hours, a greater than 3-log reduction in the total viable cell count of Candida krusei NCPF® 3876 and Pseudomonas aeruginosa NCIMB 8626 on each of the respective tested polycarbonate coupons was achieved when the coupons were exposed to the respective wound dressing according to the invention.

Examples 13 demonstrates that, within 168 hours, a greater than 3-log reduction in the total viable cell count of Staphylococcus aureus NCIMB 9518 on the respective tested polycarbonate coupon was achieved when the coupon was exposed to the respective wound dressing according to the invention.

A greater than 3-log reduction in the total viable cell count of each tested microorganism was achieved with each coupon and dressing within 168 hours.

Thus, the wound dressing of the invention demonstrates significant reduction in biofilm cell numbers for each challenge organism on both the polycarbonate coupon and within the wound dressing over 168 hours and, in many cases, within 48 hours.

The one or more embodiments are described above by way of example only. Many variations are possible without departing from the scope of protection afforded by the appended claims.

**Claims**

1. A negative pressure wound dressing (1) comprising a backing layer (7), an adhesive skin contact layer (20) and an absorbent structure wherein the adhesive skin contact layer (20) is configured to detachably adhere the dressing to a dermal surface, wherein the backing layer (7) comprises an aperture for connection to a negative pressure source, wherein the absorbent structure comprises a wound contact layer (4) arranged to contact the wound when the adhesive skin contact layer (20) is adhered to the skin adjacent the wound, further wherein the wound contact layer (4) comprises an antimicrobial agent comprising ionic silver, **characterised in that** the wound contact layer (4) comprises multiple layers each formed of carboxyalkylcellulose, or a salt thereof, fibres.

2. A negative pressure wound dressing (1) according to claim 1, wherein the carboxyalkylcellulose, or a salt thereof, fibres have a degree of substitution of between about 0.05 carboxyalkyl groups per glucose unit and about 0.50 carboxyalkyl groups per glucose unit.

3. A negative pressure wound dressing (1) according to claim 2, wherein the degree of substitution is about 0.30 carboxyalkyl groups per glucose unit.

4. A negative pressure wound dressing (1) according to claim 2 or 3, wherein the antimicrobial agent is dispersed upon the carboxyalkylcellulose, or a salt thereof, fibres.

5. A negative pressure wound dressing (1) according to any of claims 2 to 4, wherein the carboxyalkylcellulose, or a salt thereof, fibres are sodium carboxymethylcellulose fibres.

6. A negative pressure wound dressing (1) according to any preceding claim, wherein the wound contact layer (4) has a basis weight of between 160 $g/m^2$ and 480 $g/m^2$.

7. A negative pressure wound dressing (1) according to claim 1, wherein each layer of carboxyalkylcellulose, or a salt thereof, fibres has a basis weight of between 40 $g/m^2$ and 240 $g/m^2$.

8. A negative pressure wound dressing (1) according to claim 1, wherein the combined basis weight of the multiple layers of carboxyalkylcellulose, or a salt thereof, fibres is between 160 $g/m^2$ and 480 $g/m^2$.

9. A negative pressure wound dressing (1) according to claim 1, wherein the wound contact layer (4) comprises only two layers of carboxyalkylcellulose, or a salt thereof, fibres, and optionally wherein each layer has a basis weight of 100 $g/m^2$.

10. A negative pressure wound dressing (1) according to any preceding claim, wherein the ionic silver is present in the antimicrobial agent at a concentration of from 0.10 wt. % to 10.0 wt. %.

11. A negative pressure wound dressing (1) according to any preceding claim, wherein the wound contact layer (4) further comprises an antibiofilm agent.

12. A negative pressure wound dressing (1) according to claim 11, wherein the antibiofilm agent comprises a chelating agent, optionally wherein the chelating agent is ethylenediaminetetra-acetic acid (EDTA) or a salt thereof (e.g. di-sodium or calcium di-sodium salts), further optionally wherein the chelating agent is present in an amount of between 0.5 wt.% and 10 wt.%, preferably between 1 wt.% and 3 wt.%.

13. A method for manufacturing a negative pressure wound dressing according to any of claims 1 to 12 \comprising the steps:

    a. Providing an adhesive skin contact layer and a backing layer;

b. Providing a wound contact layer and an absorbent structure comprising a transmission layer and a superabsorbent layer, wherein the wound contact layer comprises an antimicrobial agent comprising ionic silver, further wherein the wound contact layer comprises multiple layers each formed of carboxyalkylcellulose, or a salt thereof, fibres; and

c. Arranging the absorbent structure between the wound contact layer and the backing layer.

14. A negative pressure wound exudate management system (400) comprising the negative pressure wound dressing (1, 420) of any of claims 1 to 12 and further comprising:

a. A source (410) of negative pressure; and
b. a conduit or tube (440, 450) for providing negative pressure to the wound dressing (1, 420) via an internal lumen (442, 452) of the conduit or tube (440, 450) and an opening in the wound dressing (1, 420).

**Patentansprüche**

1. Unterdruck-Wundverband (1), umfassend eine Trägerschicht (7), eine haftende Hautkontaktschicht (20) und eine absorbierende Struktur, wobei die haftende Hautkontaktschicht (20) ausgebildet ist, um den Verband lösbar an einer dermalen Oberfläche haftend zu halten, **wobei** die Trägerschicht (7) eine Apertur zur Verbindung mit einer Unterdruckquelle umfasst, wobei die absorbierende Struktur eine Wundkontaktschicht (4) angeordnet, um die Wunde zu kontaktieren, wenn die haftende Hautkontaktschicht (20) an die an die Wunde angrenzende Haut gehaftet ist, umfasst, wobei ferner die Wundkontaktschicht (4) ein ionisches Silber umfassendes antimikrobielles Mittel umfasst, **dadurch gekennzeichnet, dass** die Wundkontaktschicht (4) mehrere jeweils aus Carboxyalkylcellulose-, oder einem Salz davon, -Fasern gebildete Schichten umfasst.

2. Unterdruck-Wundverband (1) gemäß Anspruch 1, wobei die Carboxyalkylcellulose-, oder ein Salz davon, -Fasern einen Substitutionsgrad zwischen etwa 0,05 Carboxyalkylgruppen pro Glucoseeinheit und etwa 0,50 Carboxyalkylgruppen pro Glucoseeinheit aufweisen.

3. Unterdruck-Wundverband (1) gemäß Anspruch 2, wobei der Substitutionsgrad etwa 0,30 Carboxyalkylgruppen pro Glukoseeinheit beträgt.

4. Unterdruck-Wundverband (1) gemäß Anspruch 2 oder 3, wobei das antimikrobielle Mittel auf den Carboxyalkylcellulose-, oder einem Salz davon, -Fasern dispergiert ist.

5. Unterdruck-Wundverband (1) gemäß einem der Ansprüche 2 bis 4, wobei die Carboxyalkylcellulose-, oder ein Salz davon, -Fasern Natriumcarboxymethylcellulose-Fasern sind.

6. Unterdruck-Wundverband (1) gemäß einem der vorhergehenden Ansprüche, wobei die Wundkontaktschicht (4) ein Flächengewicht zwischen 160 g/m$^2$ und 480 g/m$^2$ aufweist.

7. Unterdruck-Wundverband (1) gemäß Anspruch 1, wobei jede Schicht aus Carboxyalkylcellulose-, oder einem Salz davon, -Fasern ein Flächengewicht zwischen 40 g/m$^2$ und 240 g/m$^2$ aufweist.

8. Unterdruck-Wundverband (1) gemäß Anspruch 1, wobei das kombinierte Flächengewicht der mehreren Schichten von Carboxyalkylcellulose-, oder einem Salz davon, -Fasern zwischen 160 g/m$^2$ und 480 g/m$^2$ ist.

9. Unterdruck-Wundverband (1) gemäß Anspruch 1, wobei die Wundkontaktschicht (4) nur zwei Schichten von Carboxyalkylcellulose-, oder einem Salz davon, -Fasern umfasst, und optional, wobei jede Schicht ein Flächengewicht von 100 g/m$^2$ aufweist.

10. Unterdruck-Wundverband (1) gemäß einem der vorhergehenden Ansprüche, wobei das ionische Silber in dem antimikrobiellen Mittel in einer Konzentration von 0,10 Gew.-% bis 10,0 Gew.-% vorhanden ist.

11. Unterdruck-Wundverband (1) gemäß einem der vorhergehenden Ansprüche, wobei die Wundkontaktschicht (4) ferner ein Antibiofilm-Mittel umfasst.

12. Unterdruck-Wundverband (1) gemäß Anspruch 11, wobei das Antibiofilm-Mittel einen Chelatbildner umfasst, optional wobei der Chelatbildner Ethylendiamintetraessigsäure (EDTA) oder ein Salz davon (z. B. Dinatrium- oder Calcium-Dinatriumsalze) ist, ferner optional wobei der Chelatbildner in einer Menge zwischen 0,5 Gew.-% und 10 Gew.-%, bevorzugt zwischen 1 Gew.-% und 3 Gew.-%, vorhanden ist.

13. Verfahren zur Herstellung eines Unterdruck-Wundverbands gemäß einem der Ansprüche 1 bis 12, umfassend die Schritte:

a. Bereitstellen einer haftenden Hautkontaktschicht und einer Trägerschicht;
b. Bereitstellen einer Wundkontaktschicht und einer absorbierenden Struktur umfassend eine Transmissionsschicht und eine superabsorbierende Schicht, wobei die Wundkontaktschicht ein ionisches Silber umfassendes antimikrobielles Mittel umfasst,
wobei ferner die Wundkontaktschicht mehrere jeweils aus Carboxylalkylcellulose-, oder einem Salz davon, -Fasern gebildete Schichten umfasst; und
c. Anordnen der absorbierenden Struktur zwischen der Wundkontaktschicht und der Trägerschicht.

14. Unterdruck-Wundexsudat-Managementsystem (400), umfassend den Unterdruck-Wundverband (1, 420) nach einem der Ansprüche 1 bis 12 und ferner umfassend:

a. eine Unterdruckquelle (410); und
b. eine Leitung oder ein Rohr (440, 450) zum Bereitstellen von Unterdruck an den Wundverband (1, 420) mittels eines inneren Lumens (442, 452) der Leitung oder des Rohrs (440, 450) und einer Öffnung in dem Wundverband (1, 420).

**Revendications**

1. Un pansement (1) pour plaie à dépression comprenant une couche (7) de support, une couche (20) adhésive de contact avec le peau et une structure absorbante, dans lequel la couche (20) adhésive de contact avec la peau est configurée pour faire adhérer de manière détachable le pansement à une surface dermique, dans lequel la couche (7) de support comprend une ouverture de communication avec une source de dépression, dans lequel la structure absorbante comprend une couche (4) de contact avec une plaie agencée pour être en contact avec la plaie lorsque la couche (20) adhésive de contact avec la peau adhère à la peau au voisinage de la plaie, dans lequel en outre la couche (4) de contact avec la plaie contient un agent antimicrobien comprenant de l'argent ionique, **caractérisé en ce que** la couche (4) de contact avec la plaie comprend de multiples couches formées chacune de fibres de carboxyalcoylcellulose ou d'un sel de celle-ci.

2. Un pansement (1) pour plaie à dépression suivant la revendication 1, dans lequel les fibres de carboxyalcoylcellulose ou d'un sel de celles-ci ont un degré de substitution compris entre 0,05 groupe carboxyalcoyle par motif glucosique et environ 0,50 groupe carboxyalcoyle par motif glucosique.

3. Un pansement (1) pour plaie à dépression suivant la revendication 2, dans lequel le degré de substitution est d'environ 0,30 groupe carboxyalcoyle par motif glucosique.

4. Un pansement (1) pour plaie à dépression suivant la revendication 2 ou 3, dans lequel l'agent antimicrobien est dispersé sur les fibres de carboxyalcoylcellulose ou d'un sel de celle-ci.

5. Un pansement (1) pour plaie à dépression suivant l'une quelconque 2 à 4, dans lequel les fibres de carboxyalcoylcellulose ou d'un sel de celle-ci sont des fibres de carboxyméthylcellulose de sodium.

6. Un pansement (1) pour plaie à dépression suivant l'une quelconque des revendications précédentes, dans lequel la couche (4) de contact avec une plaie à un poids de base compris entre 160 g/m$^2$ et 480 g/m$^2$.

7. Un pansement (1) pour plaie à dépression suivant la revendication 1, dans lequel chaque couche de fibres de carboxyalcoylcellulose ou de l'un de ses sels a un poids de base compris entre 40 g/m$^2$ et 240 g/m$^2$.

8. Un pansement (1) pour plaie à dépression suivant la revendication 1, dans lequel le poids de base combiné des multiples couches de fibres de carboxyalcoylcellulose ou d'un de celle-ci est compris entre 160 g/m$^2$ et 480 g/m$^2$.

9. Un pansement (1) pour plaie à dépression suivant la revendication 1, dans lequel la couche (4) de contact avec une plaie comprend seulement deux couches de fibres de carboxyalcoylcellulose ou d'un sel de celle-ci, et dans lequel éventuellement chaque couche a un poids de base de 100 g/m$^2$.

10. Un pansement (1) pour plaie à dépression suivant l'une quelconque des revendications précédentes, dans lequel l'argent ionique est présent dans l'agent antimicrobien à une concentration allant de 0,10 % en poids à 10,0 % en poids.

11. Un pansement (1) pour plaie à dépression suivant l'une quelconque des revendications précédentes, dans lequel la couche (4) de contact avec une plaie comprend en outre un agent antibiofilm.

12. Un pansement (1) pour plaie à dépression suivant la revendication 11, dans lequel l'agent antibiofilm comprend un agent de chélation, dans lequel éventuellement l'agent de chélation est l'acide éthylènediaminetétra-acétique (EDTA) ou l'un de ses sels (par exemple des sels disodiques ou de calcium disodique), dans lequel en outre éventuellement l'agent de chélation est présent en une quantité comprise entre 0,5 % en poids et 10 % en poids, de préférence entre 1 % en poids et 3 % en poids.

13. Un procédé de fabrication d'un pansement pour plaie à dépression suivant l'une quelconque des revendications 1 à 12, comprenant les stades :

a. se procurer une couche adhésive de contact avec la peau et une couche de support ;
b. se procurer une couche de contact avec une plaie et une structure absorbante comprenant une couche de transmission et une couche super absorbante, dans lequel la couche de contact avec une plaie comprend un agent antimicrobien comprenant de l'argent ionique, dans lequel, en outre, la couche de contact avec une plaie comprend de multiples couches formées chacune de fibres de carboxyalcoylcellulose ou d'un sel de celle-ci ; et
c. mettre la structure absorbante entre la couche de contact avec une plaie et la couche de support.

14. Un système (400) à dépression de gestion de l'exsudat d'une plaie comprenant le pansement (1, 420) pour plaie à dépression de l'une quelconque des revendications 1 à 12 et comprenant en outre :

a. une source (410) de dépression ; et
b. un conduit ou un tube (440, 450) pour donner de la dépression au pansement (1, 420) pour une plaie par l'intermédiaire d'un orifice (442, 452) interne du conduit ou du tube (440, 450) et d'une ouverture dans le pansement (1, 420) pour plaie.

EP 4 577 254 B1

*Fig. 1*

*Fig. 2*

EP 4 577 254 B1

_Fig. 3_

P. Aeruginosa NCTC 8506

Fig. 4

MRSA ATCC BAA-1556

Fig. 5

*C. krusei* NCPF 3876

*Fig. 6*

Vancomycin resistant Enterococcus faecalis NCTC 12201

*Fig. 7*

Methicillin Resistant Staphylococcus aureus ATCC® BAA-1556™ (MRSA)

*Fig. 8*

Staphylococcus pyogenes NCTC 10872

*Fig. 9*

Acinetobacter baumannii NCTC 13421

*Fig. 10*

ESBL producing Klebsiella pneumoniae NCTC 13465

*Fig. 11*

Antibiotic resistant Pseudomonas aeruginosa NCTC 8506

*Fig. 12*

Aspergillus brasiliensis NCPF® 2275

*Fig. 13*

Candida krusei NCPF® 3876

*Fig. 14*

Candida krusei NCPF® 3876

*Fig. 15*

*Fig. 16*

Staphylococcus aureus NCIMB 9518

Fig. 17

Fig. 18

Pseudomonas aeruginosa NCIMB 8626

*Fig. 19*

*Fig. 20*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021146000 A1
- WO 0001425 A
- WO 2012061225 A